# EUROPEAN PATENT SPECIFICATION

(11) **EP 3 294 711 B1**
(45) Date of publication and mention of the grant of the patent: **08.01.2020**
(21) Application number: 16729373.7
(22) Date of filing: 09.05.2016
(51) Int. Cl.: C07C 403/20, A23L 33/10, A61P 25/00, A61K 31/203

(54) **STEREOSELECTIVE SYNTHESIS OF 9-CIS-13,14-DIHYDRORETINOIC ACID AND ITS ETHYL ESTERS**
STEREOSELEKTIVE SYNTHESE VON 9-CIS-13,14-DIHYDRORETINSÄURE UND DEREN ETHYLESTERN
SYNTHÈSE STÉRÉOSÉLECTIVE D'ACIDE 9-CIS-13,14-DIHYDRORÉTINOÏQUE ET DE SES ESTERS D'ÉTHYLE

(30) Priority: 08.05.2015 US 201562158634 P
(43) Date of publication of application: 21.03.2018
(73) Proprietor: University of Debrecen, 4032 Debrecen (HU); Universidade de Vigo, 36310 Vigo (Pontevedra) (ES); Université de Strasbourg, 67081 Strasbourg Cedex (FR)
(72) Inventor: KREZEL, Wojciech, 67081 Strasbourg Cedex (FR); RÜHL, Ralph, 4002 Debrecen (HU); DE LERA, Angel R, 36310 Vigo (Pontevedra) (ES)
(74) Representative: Svingor, Adam
(86) International application number: PCT/IB2016/052639
(87) International publication number: WO 2016/181288

(56) References cited:
- WO-A1-2013/134867
- WO-A2-2006/029398

## Description

Retinoid X receptors (RXRs) are ligand-activated transcription factors controlling a variety of physiological processes. The invention relates to novel enantiomer compounds which are derivatives of dihydroretinoic acid, their stereoselective synthesis, to pharmaceutical compositions containing the same.

### BACKGROUND OF THE INVENTION

Micronutrients such as vitamin A and polyunsaturated fatty acids are essential ingredients of mammalian diet and can act as bioactive molecules. Nuclear hormone receptors sense such molecular signals and accordingly regulate gene expression, thus functioning as ligand controlled transcription factors. Retinoid X receptors (RXRs) occupy a central place in nuclear receptor signaling as obligatory heterodimerization partners for several of those receptors. RXR ligands can regulate activity of some heterodimers including for example LXR-RXR or PPAR-RXR, collectively called permissive heterodimers in opposition to non-permissive heterodimers, like RAR-RXR, which cannot be activated by RXR ligands alone [1, 2].

Ligand-dependent modulations might be particularly relevant for control of various physiological events and their pathology. In particular, a number of synthetic RXR agonists are currently in clinical development for the treatment of cancer and metabolic diseases [3]. WO2013/134867 describes a method of improving visual function in a subject by administration of a synthetic retinal. Recent studies also showed that memory functions are affected by RXR specific ligands [4], suggesting their utility for the treatment of some neuropsychiatric or neurodegenerative disorders [4, 5, 6].

In contrast to development and use of synthetic RXR ligands, no endogenous RXR ligand has been conclusively demonstrated by previous studies [7, 8, 9]. Among the potential RXR ligands, 9-*cis*-retinoic acid (9CRA) [10], an isomer of all-*trans*-retinoic acid (ATRA), was either undetectable [11, 12, 13, 14, 15, 16] or was not present in sufficient concentrations [17] to enable RXR-mediated signaling in mammalian organisms. Docosahexaenoic acid (DHA), shown to bind and transactivate RXRs under pharmacological conditions [7, 18, 19] can be detected in the brain mainly in esterified form contributing to e.g. structural components of the cell while the pool of this fatty acid available for RXR activation remains too low [20, 21]. Finally, phytanic acid [22, 23] also suggested to bind RXR was shown not to be physiologically relevant.

In the context, inventors focused on novel endogenous retinoid, which is detectable in mice at concentration sufficient to displays RXR agonistic activities *in vitro* and *in vivo.*

### SUMMARY OF THE INVENTION

Surprisingly, inventors prepared enantiomerically pure or pure enantiomer of 9-*cis*-13,14-dihydroretinoic acid (9CDHRA) with a novel synthesis method.

Surprisingly, inventors characterized 9-*cis*-13,14-dihydroretinoic acid (9CDHRA) as the first endogenous and physiologically relevant retinoid which acts as RXR ligand in mammals.

The present invention concerns, in a first aspect, the compound of formula (I) and to a method for preparing same. The invention also relates to pharmaceutical compositions comprising the compounds according to the invention in a pharmaceutically acceptable carrier.

The present invention concerns, in another aspect, pharmaceutical composition containing enantiomerically pure 9-*cis*-13,14-dihydroretinoic acid (9CDHRA). The invention also relates to pharmaceutical compositions comprising the compounds according to the invention in a pharmaceutically acceptable carrier. The invention also describes a pharmaceutical composition for use in a therapeutic method, in particular for the treatment of a psychiatric disorder/disease.

More specifically, the invention relates to a method for the preparation of a compound selected from the group consisting of R or S enantiomer of 9-cis-13,14-dihydroretinoic acid (R-9CDHRA or S-9CDHRA) or esters thereof according to general formula I [(R)-I and/or (S)-I], a solvate and, if appropriate, a salt thereof, said method comprising 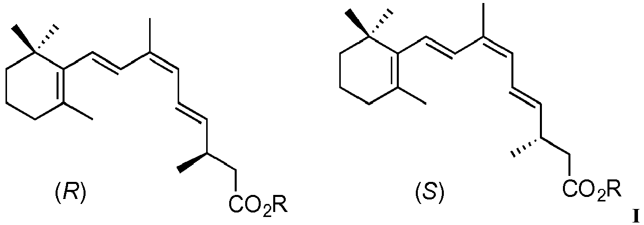 wherein in said formula R is selected from H or Ethyl;
- providing the respective 9Z,11E geometric isomer of R or S enantiomer (preferably enantiopure) trienyliodide of Formula **3** [(R)-I and/or (S)-I] 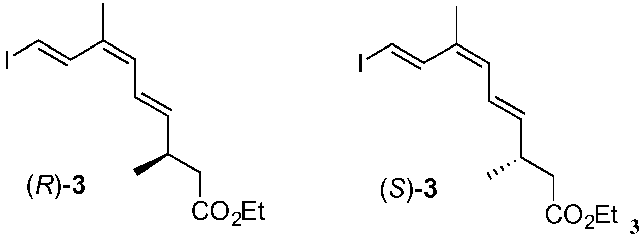
- reacting, respectively, said R or S enantiomer (preferably) enantiopure trienyliodide of Formula **3** with boronic acid of Formula **2** by Suzuki coupling to obtain said compound as R or S ethyl-9-cis-13,14-dihydroretinoate of Formula (I); 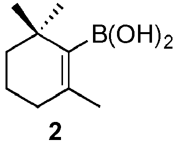
- optionally saponifying said R or S ethyl-9-cis-13,14-dihydroretinoate to obtain said compound as R or S 9-cis-13,14-dihydroretinoic acid, respectively; and
- optionally forming said compound into a solvate or, if appropriate, salt thereof.

Preferably, the R or S enantiomer, preferably enantiopure, trienyliodide of Formula **3** is prepared from the enantiopure stannane of Formula **9** [(R)-9 and/or (S)-9] 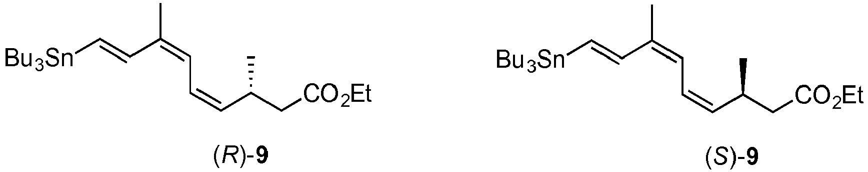 with a solution of iodine in solvent, preferably CH₂Cl₂, via Sn-I exchange and iodine-promoted isomerization of the 9Z,11Z diene to the desired 9Z,11E geometric isomer.

Preferably, said method further comprises the steps of
(a) transforming the stannyldienol of Formula **5** 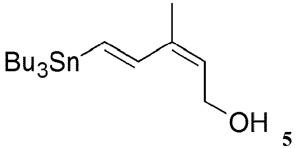 by Mitsunobu reaction with the corresponding thiol into benzothiazolyl allyl sulfide having Formula **6** 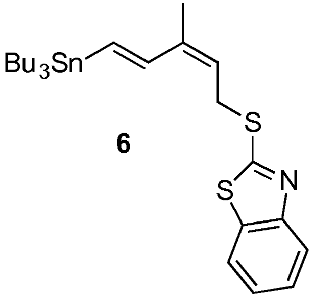
(b) oxidizing the benzothiazolyl allyl sulfide having Formula 6 to the corresponding sulfone having Formula **7** 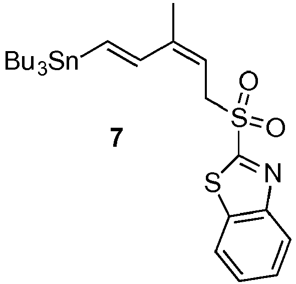 with hydrogen and a peroxymolybdate (VI) reagent, preferably at - 10 °C;
- (c) reacting the sulfone having formula **7** with (preferably enantiopure) R or S aldehyde **8** [(R)-8 and/or (S)-8], (preferably 1.7 equivalents thereof), respectively, 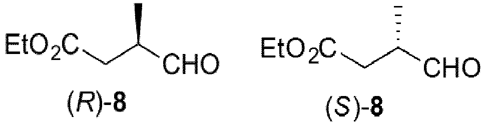
in the presence of base, preferably excess of base or slight excess of base, by Julia-Kocienski olefination, to obtain the stannane of Formula **9** [(R)-9 and/or (S)-9].

In a preferred embodiment all steps of the methods as defined above are carried out.

Preferably the compound of the invention is obtained as an enantiopure or enantiomerically pure compound.

Highly preferably, in the method of the invention, an R enantiomer of 9-cis-13,14-dihydroretinoic acid, a solvate and/or a salt thereof is prepared,
wherein Formula I is 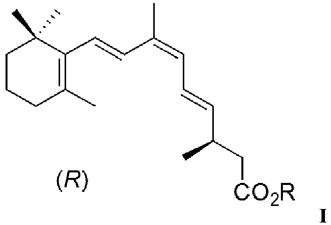 wherein in said formula R is H;
the compound of Formula 3 is 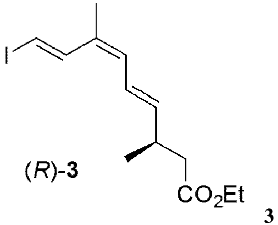 wherein the stannane of Formula **9** is of R configuration

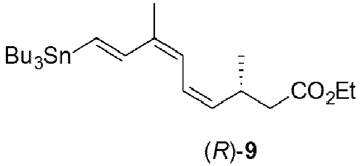

Preferably the sulfone having formula **7** is reacted with enantiopure aldehyde R of Formula **8,** respectively, 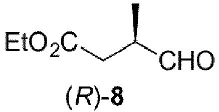 in the presence of base, to obtain the stannane of Formula **9.**

In a preferred embodiment, when an R enantiomer of 9-cis-13,14-dihydroretinoic acid, a solvate and/or a salt thereof is prepared, all steps of the methods as defined above are carried out.

Preferably the sulfone having formula **7** is reacted with 1.7 equivalents of enantiopure aldehyde R or S aldehyde **8.**

Preferably the compound of the invention is obtained as an enantiopure or enantiomerically pure compound.

In a further aspect the invention relates to an enantiomer of compound of 9-*cis*-13,14-dihydroretinoic acid or an ester precursor thereof, wherein said compound is selected from the group consisting of
(*R*)-9-*cis*-13,14-dihydroretinoic acid,
(*S*)- 9-*cis*-13,14-dihydroretinoic acid
(9Z,13R)-ethyl-13,14-dihydroretinoate((R)-4) and
(9Z,13R)-ethyl 13,14-dihydroretinoate ((S)-4), and
solvates, solid forms and, if appropriate, salts thereof.

In a further aspect the invention relates to a dietary supplement or a nutraceutical or a functional food; or relates to pharmaceutical composition comprising the enantiomer of a compound selected from these compounds, solvates, solid forms and, if appropriate, salts thereof, in a pharmaceutically acceptable carrier. Preferably said compound is obtainable or obtained according to the invention.

Preferably, the invention also relates to the enantiomer compound of 9-*cis*-13,14-dihydroretinoic acid wherein said compound is selected from the group consisting of
*(R)-9-cis-13,14-dihydroretinoic* acid,
(*S*)-9-*cis*-13,14-dihydroretinoic acid, and
solvates, solid forms and salts thereof, preferably for use in a therapeutic method.

Preferably in an embodiment said enantiomer compound of 9-*cis*-13,14-dihydroretinoic acid is for use in the treatment of a psychiatric disorder.

Preferably in an embodiment said enantiomer compound of 9-*cis*-13,14-dihydroretinoic acid is for use in the treatment of memory impairment or depression, wherein preferably said memory impairment is working memory impairment.

Preferably in an embodiment said enantiomer compound of 9-*cis*-13,14-dihydroretinoic acid is for use in enhancing memory performance, wherein preferably said memory is working memory.

Preferably in an embodiment said enantiomer compound of *9-cis-13,14-dihydroretinoic* acid is for use in the prevention and/or treatment of impaired cognitive functions or impaired learning.

Preferably in an embodiment said enantiomerically pure compound of 9-*cis*-13,14-dihydroretinoic acid is for use in the treatment of depression.

In a further aspect the invention relates to a dietary supplement or a nutraceutical or a functional food; or relates to pharmaceutical composition comprising an enantiomerically pure compound selected from these compounds, solvates, solid forms and, if appropriate, salts thereof, in a pharmaceutically acceptable carrier. Preferably said compound is obtainable or obtained according to the invention.

Highly preferably, the invention relates to an enantiomerically pure (R)-9-cis-13,14-dihydroretinoic acid or a solvate, solid form or salt thereof. In a further aspect the invention relates to a dietary supplement or a nutraceutical or a functional food; or relates to pharmaceutical composition comprising an enantiomerically pure (*R*)-9-*cis*-13,14-dihydroretinoic acid, solvate, solid form or salt thereof, in a pharmaceutically acceptable carrier. Preferably said compound is obtainable or obtained according to the invention.

In a highly preferred embodiment the invention relates to an enantiomerically pure (*R*)-9*-cis*-13,14-dihydroretinoic acid for use in the prevention and/or treatment of memory impairment or for use in enhancing memory performance.

In a highly preferred embodiment the invention relates to an enantiomerically pure (*R*)-9-*cis*-13,14-dihydroretinoic acid for use in the prevention and/or treatment of working memory impairment or for use in enhancing working memory performance.

In a highly preferred embodiment the invention relates to an enantiomerically pure (*R*)-9-*cis*-13,14-dihydroretinoic acid for use in the prevention and/or treatment of impaired cognitive functions or impaired learning.

In a highly preferred embodiment the invention relates to an enantiomerically pure (*R*)-9-*cis*-13,14-dihydroretinoic acid for use in the treatment of depression.

The invention also relates to a functional food or a dietary supplement comprising one or more enantiomerically pure compound(s) according to the invention.

In a preferred embodiment the enantiomer compound of the invention is an enantiopure or enantiomerically pure compound or obtained as an enantiopure or enantiomerically pure compound.

In a particularly preferred embodiment in the solutions of the invention comprising is to be understood as consisting essentially of or particularly highly preferably consisting of.

### BRIEF DESCRIPTION OF THE DRAWINGS

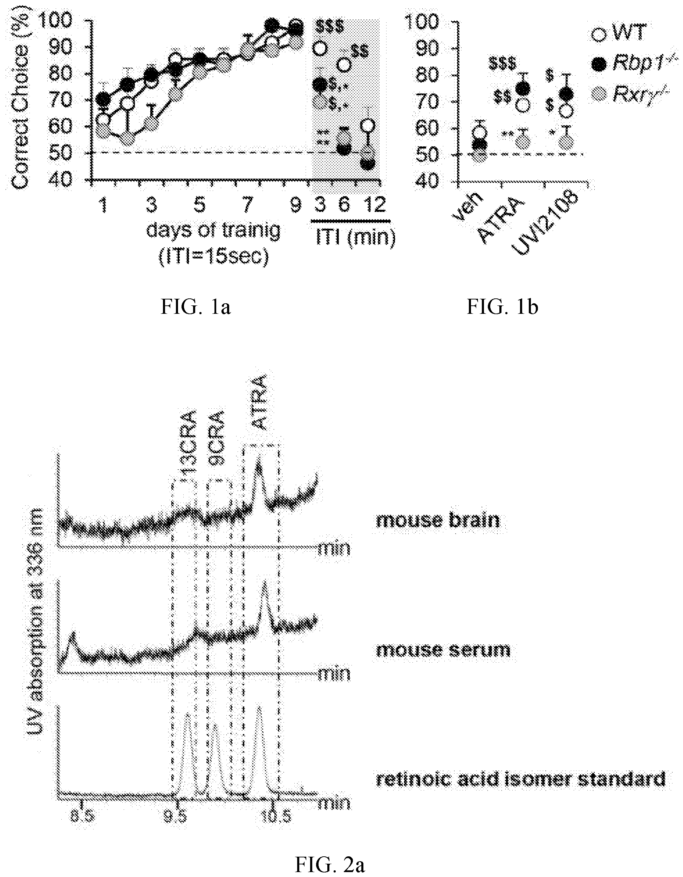

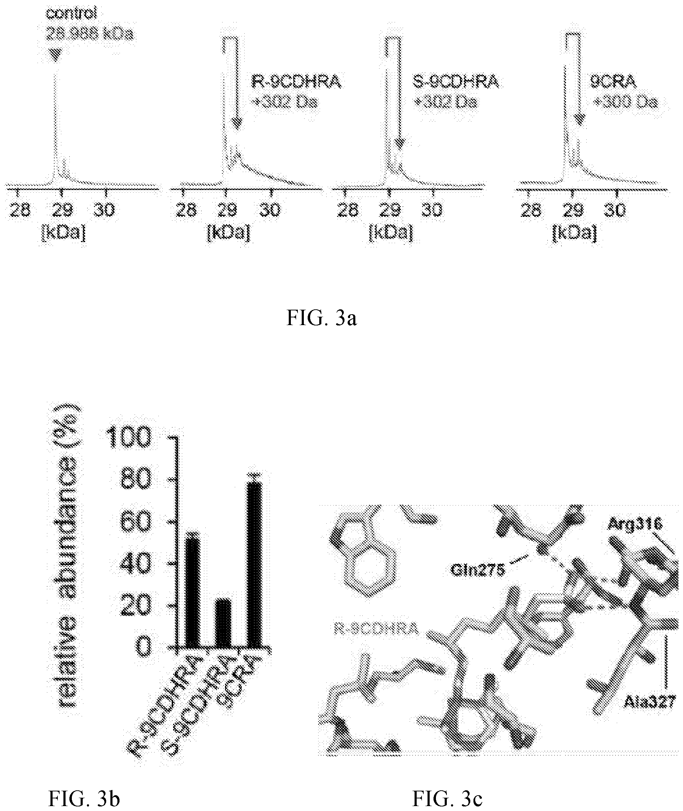

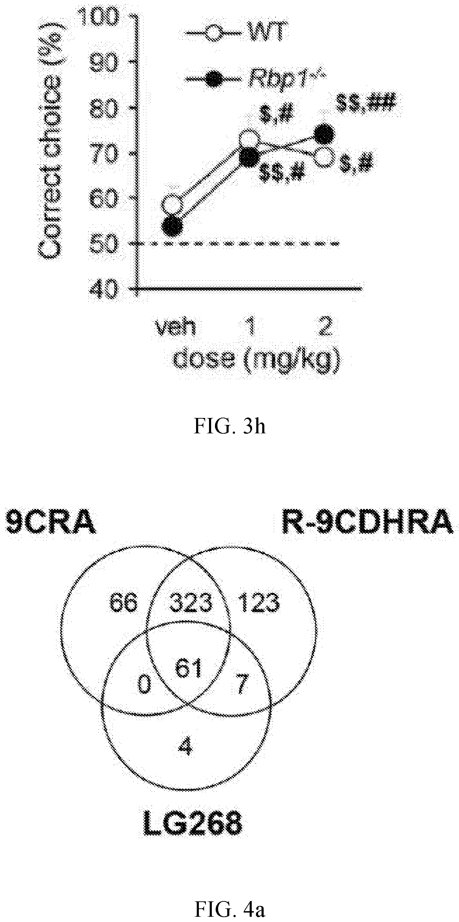

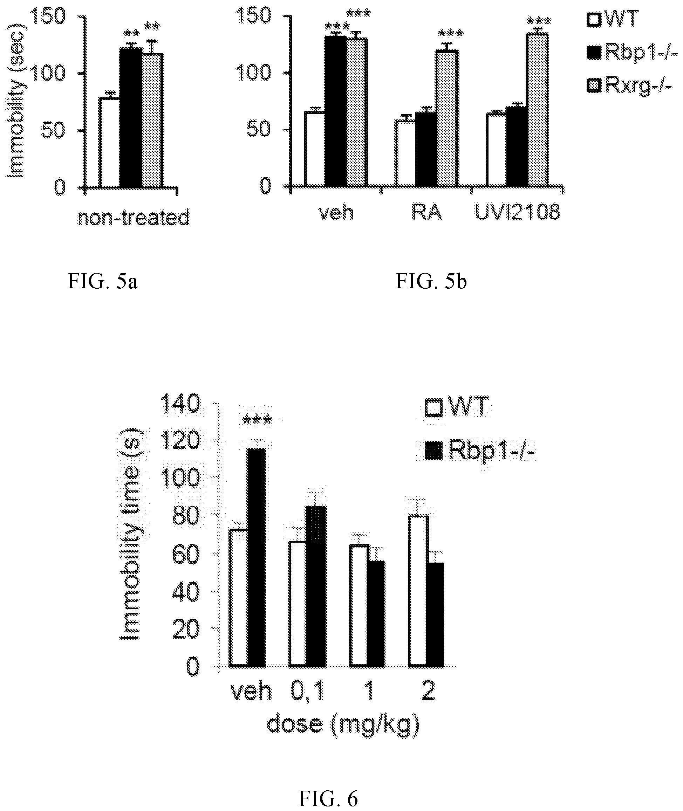

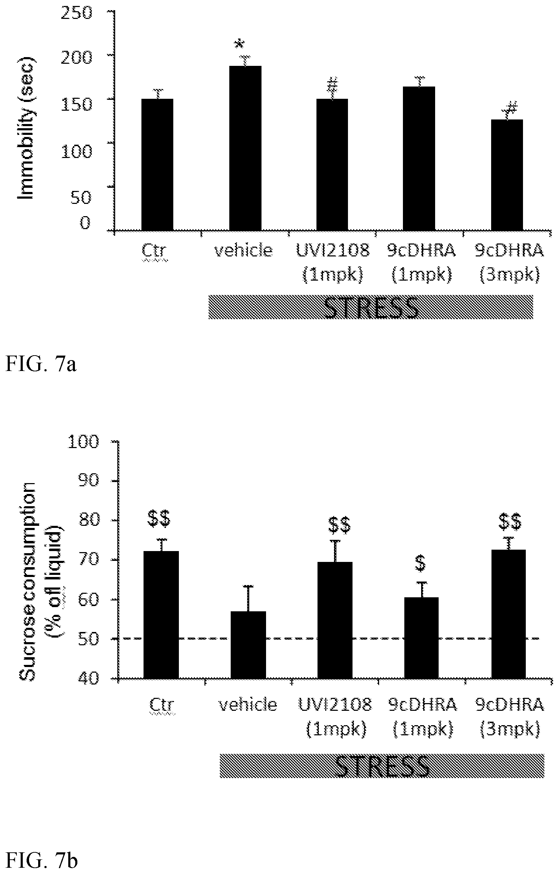

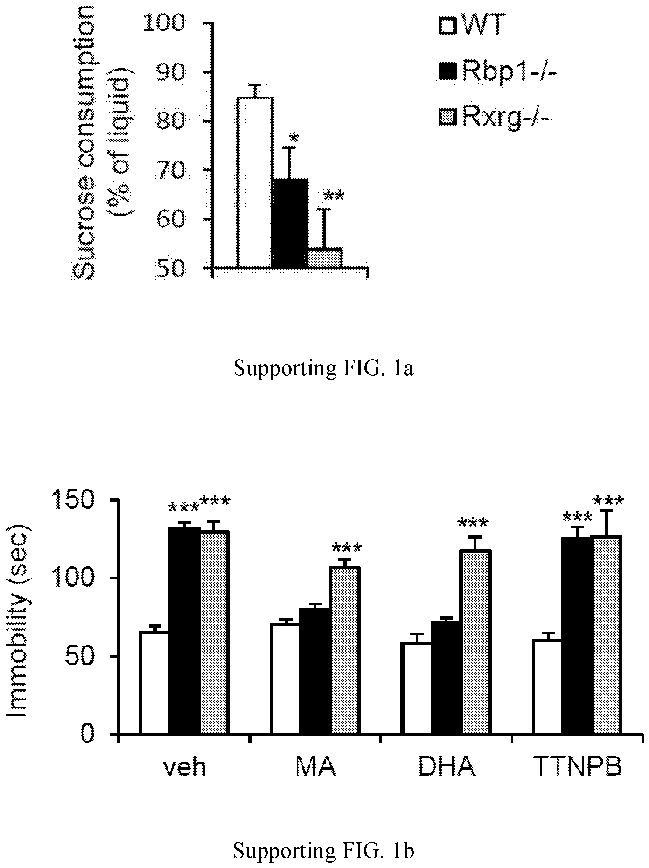

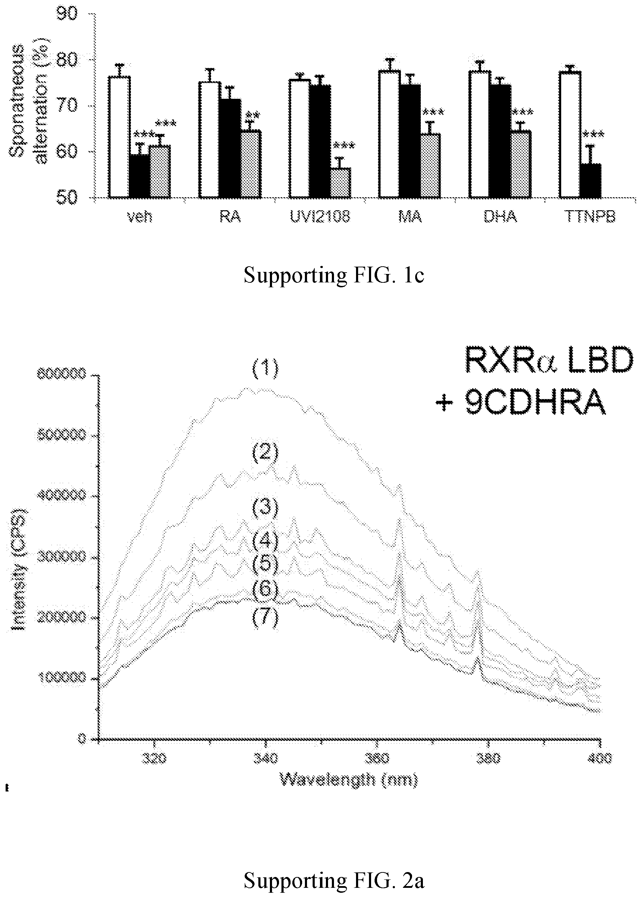

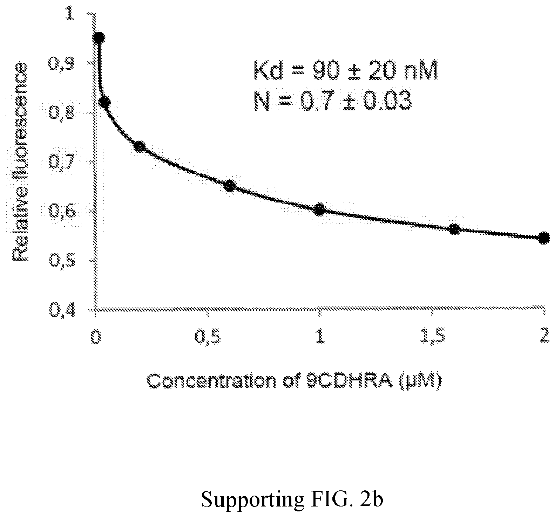

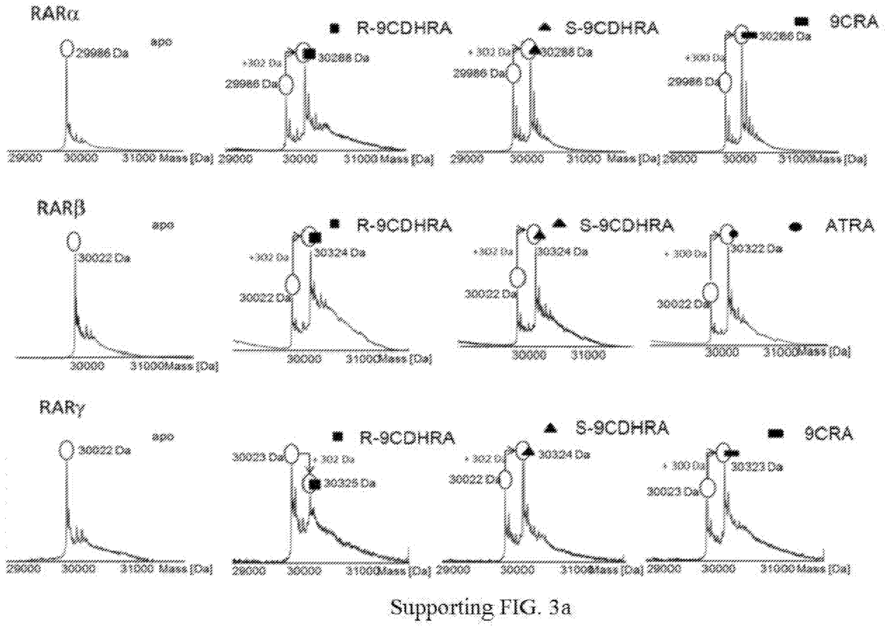

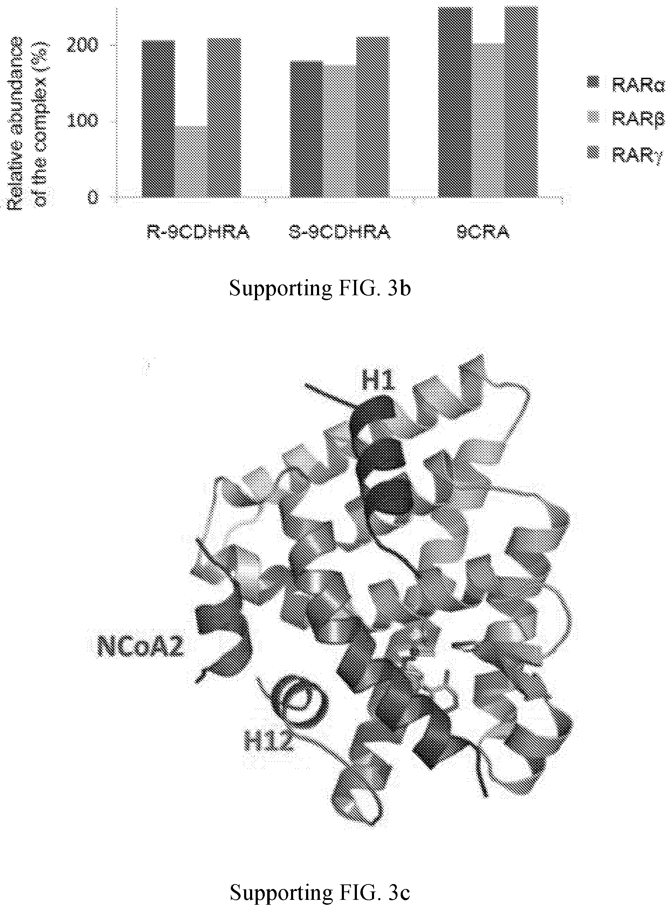

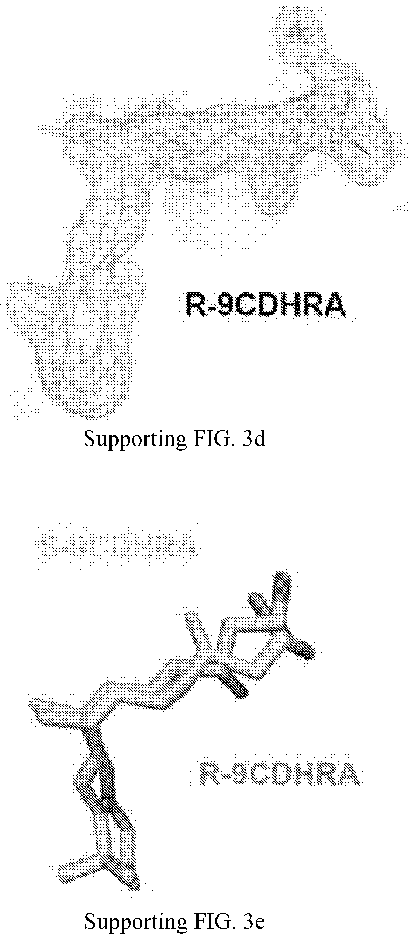

**Figs. 1a-1b****. Compromised RXR signaling in Rbp1-/- mice.**
   (a) Rbpl-/- (n=8) and Rxry-/- (n=7) mice acquired working memory DNMTP task similarly to WT (n=8) mice (F[8,160]=14, p<0.001; ANOVA on repeated measures) when trained with 15 sec inter-trial intervals (ITI), but showed forgetting when tested at 3, and 6 min ITIs, which was more rapid than 12 or 18 min in WT mice (indicated in the graph as 12 min ITI).
   (b) RXR agonists improved working memory performance in Rbpl-/- mice tested at 6 min ITI or WT mice at 12 or 18 min ITIs, but was inactive in Rxry-/- mice at ITI of 6 min. Statistical differences identified with PLSD Fischer test were indicated as: *, p<0.05; **, p<0.01 as compared to WT controls in respective groups; $, p<0.05; $$, p<0.01; $$$, p<0.001 in comparison with 50% of chance level.
**Figs. 2a-2c****. 9CDHRA is present in mouse serum and liver.**
   (a) Elution profiles of WT mouse serum (n=7) and brain (n=3) samples in comparison with three retinoic acid standards (*all-trans, 13-cis* and 9-*cis*-retinoic acid; ATRA, 13CRA, 9CRA respectively) allow identification of ATRA but not 9CRA.
   (b) 9-*cis*-13,14-diyhydroretinoic acid (9CDHRA) is present in mouse serum (n=7) and liver (n=7) samples as determined by co-elution with a mixture of 9CDHRA and all-*trans*-13,14-dihydroretinoic acid (ATDHRA) standard solution and confirmed by MS-MS (303->207 m/z) and DAD (290 nm) detection.
   (c) Significant reduction of 9CDHRA, but not ATDHRA levels in serum brain and liver of Rbpl-/- animals (n=8) as compared to WT mice (n=8). All the error bars represent S.E.M.
**Figs. 3a-3h****. 9CDHRA binds and transactivates RXR in vitro, and displays RXR agonist-like activities *in vivo.***
   (a) ESI mass spectra of hRXRα LBD protein after incubation with a 5-fold molar excess of 9CRA, (R)-9CDHRA and (*S*)-9CDHRA.
   (b) Distribution plot of the percentage of bound for hRXRα.
   (c) Close-up view showing the ligand-binding pocket (LBP) of RXRα bound to (*R*)-9CDHRA (in grey). Dashed lines indicate hydrogen bonds.
   (d) Superposition of the RXRα ligand-binding pocket bound by (*R*)-9CDHRA (grey) and 9CRA (cyan, PDB code 1XDK).
   (e) Transcriptional activation of RXRα by (*R*)-9CDHRA and (*S*)-9CDHRA in comparison to 9CRA (10⁻⁵ - 10⁻⁹M) in RXR-reporter COS1 cells.
   (f) RXR-antagonist LG101208 (LG) diminishes 9CDHRA induced RXR-mediated signaling.
   (g) Transcriptional activation of RAR-RXR heterodimers by (R)- and (*S*)-9CDHRA in comparison to ATRA in RAR-RXR-reporter COS1 cells.
   (h) Increasing doses of (*R*)-9CDHRA reversed working memory deficits in Rbpl-/- mice and showed pro-mnemonic activity in WT mice (n=8/group) in DNMTP task when tested at minimal ITI, at which mice performed at chance level (50%) and which was 6min for the Rbpl-/- or 12min for WT mice. ttt: ATRA at concentration 10-5 M was cytotoxic.*, p<0.05. #, p<0.05; ##, p<0.01 as compared to vehicle treatment in the same group; $, p<0.05; $$, p<0.01; one group student t-test for comparison with performance at chance level of 50%. All the error bars represent S.E.M.
**Figs. 4a-4c****. Molecular evidence for 9CDHRA selective activation of RXRs.**
   (a) Significant overlap between global transcriptional changes induced by (*R*)-9CDHRA (10-5M), 9CRA (10-6M) or a synthetic RXR agonist (LG268; 10-7M) revealed by DNA microarray analyses in differentiating monocyte-derived human dendritic cells.
   (b) Scatter plot comparison of fold-changes for transcripts altered by 9CRA and (*R*)-9CDHRA treatments.
   (c) (S)- and (*R*)-9CDHRA, similarly to 9CRA and/or LG268 (see "RXR" columns), induced the expression of genes (see rows) identified previously as direct transcriptional targets of LXR-RXR, PPAR-RXR and RAR-RXR. Corresponding transcripts were also induced by agonists of respective RXR-heterodimer partners (see "partners" column and arrowheads): GW3965 (LXRα/β; 10⁻⁶M), RSG (PPARγ; 10⁻⁶M), GW1516 (PPARδ; 10⁻⁶M) and AM580 (RAR; 10⁻⁷M).
**Figs. 5a-5b****. Compromised RXR signaling in Rbp1-/- mice.**
   (a) Rbpl-/- (n=14) and Rxry-/- (n=11) mice display increased despair behaviour in the forced swim test as compared to WT mice (n=20).
   (b) All*-trans* retinoic acid (RA), similarly to a synthetic RXR agonist, UVI2108 (known also as BMS649) reduced significantly immobility time of Rbp1-/- mice in the forced swim test
**Fig. 6****. R-9CDHRA displays RXR agonist-like activities in vivo.** Reverse of behavioural deficits after treatments with UVI2108 and R-9CDHRA.
   Increasing doses of R-9CDHRA (0.1, 1, 2 mg/kg) reduce despair behaviour in Rbpl-/- mice in the forced swim test (n=26 for vehicle groups and n=6-8 for each remaining group); Statistical differences revealed by PLSD Fisher test were indicated as: ***, p<0.001 for comparison with vehicle treated WT controls in respective group. $, p<0.05; $$, p<0.01; one group student t-test for comparison with performance at chance level of 50%. All the error bars represent S.E.M.
**Fig. 7a-7b****. R-9CDHRA** displays antidepressant effects in chronic social defeat stress model.
   (a) Social defeat stress significantly increased immobility time in the forced swim test in mice receiving (vehicle; n=12) as compared to non-stressed control (ctr; n=17) mice. 9cDHRA treatments decreased such immobility in stressed mice in a dose dependently manner (n=8 for 1mg/kg and n=6 for 3mg/kg of 9cDHRA) and to a similar extent as synthetic RXR agonists UVI2108 (n=12).
   (b) Sucrose preference deficit induced by social defeat stress was prevented by UVI2108 or by 9cDHRA treatments. Statistical differences revealed by PLSD Fisher test were indicated as : *, p<0.05 when compared to control mice; #, p<0.05 as compared to vehicle treated stressed mice; $$, p<0.01, $, p<0.05 as compared to absence of sucrose preference corresponding to the value of 50% of sucrose consumption. All the error bars represent S.E.M
**Supporting** **Figs. 1a-1c****. Compromised RXR signalling in Rbp1-/- mice.**
   (a) preference for 0.8% sucrose solution was significantly reduced in RBP1-/- (n=19) but not in RXRg-/- (n=11) mice as comparable with WT mice (n=22).
   (b) increased immobility in the forced swim task can be normalised in Rbpl-/- mice using methoprene acid (MA), docosahexaenoic acid (DHA) but not TTNPB (n=10-19/group).
   (c) Working memory deficits observed in the spontaneous alternation task in the Y-maze could be normalised in Rbpl-/- mice, but not in Rxrγ -/- mice using all-trans retinoic acid (ATRA; 5mg/kg), UVI2108 (1mg/kg), MA (5mg/kg), DHA (1mg/kg), but not TTNPB (5mg/kg) (n=8-15/group). All compounds were applied 5-6 hours prior to testing and all mice were tested only one time in this task. Statistical differences revealed by PLSD Fisher test results were indicated: **, p<0.01; ***, p<0.001 as compared to WT animals in respective group.
**Supporting** **Figs. 2a-2b****. Fluorescence quenching assay.**
   (a) An example of fluorescence emission spectra for the binding of increasing amounts of 9CDHRA to RXRα LBD (1.25µM). Curves 1-7 correspond to concentration of 0, 0.2, 0.45, 0.6, 1, 1.6, 1.2µM of 9CDHRA;
   (b) Titration curve for 9CDHRA was used to calculate the Kd value of 90 ± 20nM as previously described [74]. N corresponds to number of binding sites.
**Supporting** **Figs. 3a-3e****. Binding of 9CDHRA (R and S enantiomers) to RAR LBD isotypes in non-denaturing ESI-MS assays and in silico, and crystallography analyses of 9CDHRA binding to RXR.**
(a) ESI mass spectra of hRARα (top), hRARβ (middle) and hRARγ (bottom) LBDs protein after incubation with a 5-fold molar excess of ligands;
   (b) Distribution plot of the relative proportion of percent of bound/free protein for hRAR isotypes for R and S enantiomers 9CDHRA and 9CRA.
   (c) Overall crystal structure of the RXRalpha LBD in complex with (R)-9CDHRA.
   (d) Experimental 2Fo-Fc electron density map of (R)-9CDHRA ligand contoured at 1sigma.
   (e) Modeling of the binding of (S)- 9CDHRA in RXRalpha ligand binding pocket and superposition of (S)-9CDHRA (in cyan) and (R)-9CDHRA (in green) revealed that the side chain of (S)-9CDHRA adopts slightly different position due to the inverse configuration at C13, that results in a similar interaction of the carboxyl group but requires a side chain repositioning of Phe313 (Helix H5) that should result in a lower affinity to RXR. The (S)-9CDHRA generated with Marvin program was docked in the protein structure of the (R)-9CDHRA complex.

### DETAILED DESCRIPTION OF THE INVENTION

It is to be understood that this invention is not limited to particular methods, reagents, compounds, compositions or biological systems, which can, of course, vary. It is also to be understood that the terminology used herein is for the purpose of describing particular embodiments only, and is not intended to be limiting. As used in this specification and the appended claims, the singular forms "a", "an" and "the" include plural references unless the content clearly dictates otherwise.

The compounds according to the invention relate to enantiomers (pure enantiomers or mixture provided that they are obtainable by the method of the invention), geometric isomers of same, salts, hydrates and solvates of same, solid forms of same, as well as mixtures of said forms.

"Enantiomer" is preferably understood herein as a compound of the invention as obtainable by the enantioselective preparation method of the invention, preferably as an enantiopure or enantiomerically pure compound. "Enantiopure" or "enantiomerically pure" as a compound wherein the molecules have (to the extent obtainable by the method of the invention) the same chirality. Preferably enantiopure or enantiomerically pure means optically pure, more preferably having at least 90%, preferably at least 95%, more preferably at least 98% or 99% optical purity. Highly preferably the molecules of the "enantiopure" or "enantiomerically pure" have the same chirality within limits of detection.

When the compounds according to the invention are in the forms of salts, they are preferably pharmaceutically acceptable salts. Such salts include pharmaceutically acceptable acid addition salts, pharmaceutically acceptable base addition salts, pharmaceutically acceptable metal salts, ammonium and alkylated ammonium salts. Acid addition salts include salts of inorganic acids as well as organic acids. Representative examples of suitable inorganic acids include hydrochloric, hydrobromic, hydroiodic, phosphoric, sulfuric, nitric acids and the like. Representative examples of suitable organic acids include formic, acetic, trichloroacetic, trifluoroacetic, propionic, benzoic, cinnamic, citric, fumaric, glycolic, lactic, maleic, malic, malonic, mandelic, oxalic, picric, pyruvic, salicylic, succinic, methanesulfonic, ethanesulfonic, tartaric, ascorbic, pamoic, bismethylene salicylic, ethanedisulfonic, gluconic, citraconic, aspartic, stearic, palmitic, EDTA, glycolic, p-aminobenzoic, glutamic, benzenesulfonic, p-toluenesulfonic acids, sulphates, nitrates, phosphates, perchlorates, borates, acetates, benzoates, hydroxynaphthoates, glycerophosphates, ketoglutarates and the like. Further examples of pharmaceutically acceptable inorganic or organic acid addition salts include the pharmaceutically acceptable salts listed in J. Pharm. Sci. 1977, 66, 2.

The starting compounds may be obtained commercially or may be synthesized according to standard methods.

The preparation of (*R*)-9-cis-13,14-dihydroretinoic acid (*R*)-**1** was based on the Suzuki coupling of enantiopure trienyliodide **3** and boronic acid **2** (See Scheme 1 in Example 10). The synthesis of **3** started with (Z)-stannyldienol **5** which was transformed into the benzothiazolyl allyl sulfide **6** by Mitsunobu reaction with the corresponding thiol and subsequently oxidized to sulfone **7** with H₂O₂ and a peroxymolybdate (VI) reagent. The Julia-Kocienski olefination was performed using a slight excess of base and in the excess of enantiopure aldehyde (*R*)-**8.** As anticipated from previous findings on the stereochemical outcome of the reactions of allylsulfones and aldehydes, the newly formed olefin of trienyl ester (*R*)-**9** is of Z-geometry. Treatment of the precursor stannane with a solution of iodine in CH₂Cl₂ produced the iodide (*R*)-**3** via Sn-I exchange and iodine-promoted isomerization of the 9Z,11Z diene to the desired 9*Z*,11*E* geometric isomer. Geometric isomers can be confirmed by NOE experiments.

The Suzuki reaction of freshly prepared boronic acid **2** and dienyl iodide (*R*)-**3**, followed by immediate work-up resulted in ethyl (*R*)-9-*cis*-13,14-dihydroretinoate (*R*)-**4**. Saponification of (*R*)-**4** provided the desired carboxylic acid (*R*)-**1** without detectable loss of stereochemical integrity.

Following the general scheme, enantiomer (*S*)-**1** was also prepared with similar efficiency.

The preparation of the compounds of the invention is described in more detail in Example 10.

The invention also relates to pharmaceutical composition or dietary supplement comprising a compound to the invention.

The invention also relates to pharmaceutical composition comprising a compound to the invention and a pharmaceutical acceptable carrier.

The term "dietary supplement" refers to a composition intended to provide nutrients that may otherwise not be consumed in sufficient quantities.

"Nutraceutical" refers to a foodstuff that provides health benefits in addition to its basic nutritional value. A nutraceutical has a physiological benefit or provide protection against physiological disorder or discomfort.

"Functional food" refers to any modified food or food ingredient that may provide a benefit or provide protection against physiological disorder or discomfort; beyond the traditional nutrients it contains.

The compounds of the invention might be used as a nutritional or dietary supplement, in a functional food composition, in a dietary supplement composition or in a nutraceutical composition. Such nutritional or dietary supplement, functional food composition, dietary supplement compositions or nutraceutical compositions have the benefit of preventing, reversing and/or alleviating memory loss, in particular working or short term memory impairment, particularly memory loss without the administration of a pharmaceutical, i.e. in a non-medical way and/or have the benefit of preventing, reversing and/or alleviating learning impairment and/or decline of cognitive functions. By non-medical a treatment is meant for the purposes of the application wherein the normal body functions are to be maintained wherein the condition is defined herein is non-pathological or has not reached a pathological level. Preferably said dietary supplement or neutraceutical composition is administered whereas no pharmaceutical compositions are or to be administered. For example, in non-medical treatment a nutritional or dietary supplement, a functional food composition, a dietary supplement composition or a nutraceutical composition is used to prevent, reverse and/or alleviate memory loss, learning impairment and/or decline of cognitive functions in a condition which is still not or cannot be considered as an illness.

The term "carrier" refers to a diluent, adjuvant, excipient, stabilizer, or vehicle with which the agent is formulated for administration. Pharmaceutical carriers can be sterile liquids, such as water and oils, including those of petroleum, animal, vegetable or synthetic origin, such as peanut oil, soybean oil, mineral oil, sesame oil, and the like. Water is a typical carrier when the pharmaceutical composition is administered intravenously. Saline solutions and aqueous dextrose and glycerol solutions can also be employed as liquid carriers, particularly for injectable solutions. Suitable pharmaceutical excipients include starch, glucose, lactose, sucrose, gelatin, malt, rice, flour, chalk, silica gel, sodium stearate, glycerol monostearate, talc, sodium chloride; dried skim milk, glycerol, propylene, glycol, water, ethanol, and the like. The composition, if desired, can also contain minor amounts of wetting or emulsifying agents, or pH buffering agents.

Pharmaceutical compositions can take the form of solutions, suspensions, emulsion, tablets, pills, capsules, powders, sustained-release formulations, and the like. The composition can be formulated as a suppository, with traditional binders and carriers such as triglycerides.

In one embodiment, the agent is formulated in accordance with routine procedures as a pharmaceutical composition adapted for intravenous administration to human beings. Typically, compositions for intravenous administration are solutions in sterile isotonic aqueous buffer. Where necessary, the composition can also include a solubilizing agent. Generally, the ingredients are supplied either separately or mixed together in unit dosage form. For example, as a dry lyophilized powder or water-free concentrate in a hermetically sealed container such as an ampoule or sachette, indicating the quantity of active agent. Where the composition is to be administered by infusion, it can be dispensed with an infusion bottle containing sterile pharmaceutical grade water or saline. Where the composition is administered by injection, an ampoule of sterile water for injection or saline can be provided so that the ingredients can be mixed prior to administration.

Suitable oral dosage forms include, for example, tablets, pills, sachets, or capsules of hard or soft gelatin, methylcellulose or of another suitable material easily dissolved in the digestive tract. Suitable nontoxic solid carriers can be used which include, for example, pharmaceutical grades of mannitol, lactose, starch, magnesium stearate, sodium saccharin, talcum, cellulose, glucose, sucrose, magnesium carbonate, and the like. (See, e.g., Remington "Pharmaceutical Sciences", 17 Ed., Gennaro (ed.), Mack Publishing Co., Easton, Pennsylvania, 1985.)

The doses of the agents can be suitably selected depending on the clinical status, condition and age of the subject, dosage form and the like. In the case of eye drops, an agent can be administered, for example, from about 0.01 mg, about 0.1 mg, or about 1 mg, to about 25 mg, to about 50 mg, to about 90 mg per single dose. Eye drops can be administered one or more times per day, as needed. In the case of injections, suitable doses can be, for example, about 0.0001 mg, about 0.001 mg, about 0.01 mg, or about 0.1 mg to about 10 mg, to about 25 mg, to about 50 mg, or to about 90 mg of the agent, one to four times per week. In other embodiments, about 1.0 to about 30 mg of agent can be administered one to three times per week.

Methods of introduction include but are not limited to intradermal, intramuscular, intraperitoneal, intravenous, subcutaneous, intranasal, intraocular, epidural and oral routes. The agents can be administered by any convenient route such as, for example, by infusion or bolus injection, by absorption through epithelial or mucocutaneous linings (e.g., oral mucosa, rectal and intestinal mucosa), and the like, and can be administered together with other functionally active agents. Administration can be systemic or local. In addition, it can be desirable to introduce enantiomerically pure 9-*cis*-13,14-dihydro-retinoic acid or derived into the target tissue by any suitable route, including intravenous and intrathecal injection.

The compounds and the pharmaceutical compositions according to the invention may be used in a therapeutic method.

The compounds and the pharmaceutical compositions according to the invention may be used in a therapeutic method for the treatment of disease involving retinoid A receptors and retinoid X receptors.

The compounds and the pharmaceutical compositions according to the invention may be used in a therapeutic method, in particular for the treatment of psychiatric or a mental disorder/disease.

The term "psychiatric disorder" refer to psychiatric disorders include obsessive-compulsive disorder, post-traumatic stress disorder, anxiety, panic attacks, schizophrenia, schizoaffective disorders, depression, mania, manic-depression (bipolar disorder), apathy, delirium, phobias, amnesia, eating disorders (e.g., bulimia, anorexia), and the like. In one embodiment, the psychiatric disorders include obsessive-compulsive disorder, post-traumatic stress disorder, panic attacks, schizophrenia, schizoaffective disorders, depression, mania, manic-depression (bipolar disorder), apathy, delirium, phobias, amnesia, and eating disorders (e.g., bulimia, anorexia). In another embodiment, the psychiatric disorders include obsessive-compulsive disorder, schizophrenia, schizoaffective disorders, depression, mania, manic-depression (bipolar disorder), apathy, delirium, and phobias. In another embodiment, the psychiatric disorders include obsessive-compulsive disorder, schizophrenia, schizoaffective disorders, depression, mania, and manic-depression (bipolar disorder). Preferably or in particular the term "psychiatric disorder" as used herein refer to and will be understood by the skilled person as "mental disorders" as described in sections F06-F50 of WHO International Statistical Classification of Diseases and Related Health Problems 10^{th} Revision. In a preferred embodiment neurodegenerative disorders are excluded from the scope of psychiatric disorders or mental disorders.

"Working memory or synonym short-term memory" characterizes the storage, retention and recall of the information for a short interval of time spanning from some minutes to several hours, optionally days.

"Memory loss" refers to a reduction in the ability to store, retain and/or especially to recall information including past experiences, knowledge and thoughts.

The term "depression" or "depressive disorder" or "mood disorder" refers to a medical field that can be understood by the skilled practitioner. A "mood disorder" refers to disruption of feeling tone or emotional state experienced by an individual for an extensive period of time. Mood disorders include major depression disorder (i.e., unipolar disorder), mania, dysphoria, bipolar disorder, dysthymia, cyclothymia and many others. See, e.g., Diagnostic and Statistical Manual of Mental Disorders, Fourth Edition, (DSM IV). A "Major depression disorder," "major depressive disorder," or "unipolar disorder" refers to a mood disorder involving any of the following symptoms: persistent sad, anxious, or "empty" mood; feelings of hopelessness or pessimism; feelings of guilt, worthlessness, or helplessness; loss of interest or pleasure in hobbies and activities that were once enjoyed, including sex; decreased energy, fatigue, being "slowed down"; difficulty concentrating, remembering, or making decisions; insomnia, early-morning awakening, or oversleeping; appetite and/or weight loss or overeating and weight gain; thoughts of death or suicide or suicide attempts; restlessness or irritability; or persistent physical symptoms that do not respond to treatment, such as headaches, digestive disorders, and chronic pain. Various subtypes of depression are described in, e.g., DSM IV.

Those disorders known to respond positively to treatment with drugs classified as SSRIs are considered for the description of this invention as being related to depression. Such disorders are for example anxiety disorders, such as panic disorder, obsessive-compulsive disorder, posttraumatic stress disorder, chronic pain syndromes or bipolar disorder.

The compounds and/or the pharmaceutical compositions according to the invention may be used in a therapeutic method, in particular for the treatment of impaired cognitive functions and/or impaired learning.

The compounds and/or the pharmaceutical compositions according to the invention may be used in a therapeutic method, in particular for the treatment of memory impairment (memory loss), in particular working memory impairment, e.g. defects and loss, or short term memory impairment, e.g. defects and loss, like amnesia, etc. Memory impairment, impaired learning abilities and impaired cognitive functions may be associated with a mental disorder, such as mild cognitive disorder, amnestic syndrome, memory and cognitive deficiencies in schizophrenia and mood disorders, such as bipolar affective disorder and depression, stress related and anxiety disorders, such as partial and complete amnesia, dissociative amnesia.

Memory impairment, impaired learning abilities and impaired cognitive functions may be associated with a cerebrovascular disease, such as vascular dementia, inracerebral haemorrhage, intracranial haemorrhage, cerebral infarction, stroke, stenosis of cerebral arteries, cerebral atherosclerosis, cerebral aneurysm, cerebral arthritis.

Memory impairment, impaired learning abilities and impaired cognitive functions may be associated with and form a symptom of a inflammatory disease of the central nervous system, such as meningitis, (herpes) encephalitis and myelitis.

Several psychoactive substances and medications are known to cause memory loss, impaired learning abilities and impaired cognitive functions, such as tricyclic antidepressants, dopamine agonists, antihistamines, benzodiazepines, statins, beta blockers, barbiturates, opioids, THC, alcohol, etc.

The compounds and/or the pharmaceutical compositions according to the invention are especially useful in the prevention and/or treatment of short term memory impairment. The compounds and/or the pharmaceutical compositions according to the invention are particularly useful in improving working memory performance or reducing working memory loss.

The compounds and/or the pharmaceutical compositions according to the invention are useful in the treatment of depression, such as mild, moderate and severe depressive episodes as well as the depressive phase of bipolar disease, cyclothymia or dysthymia, mixed anxiety-depression disorder, depression or depressive episode associated with other diseases, such as schizophrenia, cancer, metabolic diseases, etc.

According to a specific embodiment, the invention also relates to a kit that is suitable for the treatment by the methods described above. These kits comprise a composition containing the compound of the invention in the dosages indicated above and a second composition containing an mood stabilizers, such as, for example, carbamazepine, divalproex, gabapentin, lamotrigine, lithium, olanzapine, oxycarbazepine, quetiapine, valproate, valproic acid, verapamil, and equivalents and pharmaceutically active isomer(s) and metabolite(s) thereof.

These kits comprise a composition containing the compound of the invention in the dosages indicated above and a second composition containing an antidepressants, such as, for example, agomelatine, amitriptyline, amoxapine, bupropion, citalopram, clomipramine, desipramine, doxepin, duloxetine, escitalopram, fluvoxamine, fluoxetine, gepirone, imipramine, ipsapirone, isocarboxazid, maprotiline, mirtazepine, nortriptyline, nefazodone, paroxetine, phenelzine, protriptyline, ramelteon, reboxetine, robalzotan, selegiline, sertraline, sibutramine, thionisoxetine, tranylcypromaine, trazodone, trimipramine, venlafaxine, and equivalents and pharmaceutically active isomer(s) and metabolite(s) thereof.

These kits comprise a composition containing the compound of the invention in the dosages indicated above and a second composition containing an mood stabilizers, anxiolytics, such as, for example, alnespirone, azapirones, benzodiazepines, and barbiturates, such as, for example, adinazolam, alprazolam, balezepam, bentazepam, bromazepam, brotizolam, buspirone, clonazepam, clorazepate, chlordiazepoxide, cyprazepam, diazepam, estazolam, fenobam, flunitrazepam, flurazepam, fosazepam, lorazepam, lormetazepam, meprobamate, midazolam, nitrazepam, oxazepam, prazepam, quazepam, reclazepam, suriclone, tracazolate, trepipam, temazepam, triazolam, uldazepam, zolazepam, and equivalents and pharmaceutically active isomer(s) and metabolite(s) thereof.

Below the invention is further illustrated by non-limiting examples.

### EXAMPLES:

### Example 1: Animals:

Rbpl-/- and Rxrγ -/- mutants as well as wild type (WT) control mice were raised on a mixed genetic background (50% C57BL/6J and 50% 129SvEms/j; bred for more than 10 generations) from heterozygous crosses as described [24,33], and tested at the age of 3-6 months. All mice were housed in groups of 4-5 mice per cage in a 7am-7pm light/dark cycle in individually ventilated cages (Techniplast, Italy). Food and water were freely available. All experiments were carried out in accordance with the European Community Council Directives of 24 November 1986 (86/609/EEC) and in compliance with the guidelines of CNRS and the French Agricultural and Forestry Ministry (decree 87848).

### Example 2: Behavioral procedures:

All behavioral tests were carried out in the Institute Clinique de la Souris (http://www.ics-mci.fr/) according to standard operating procedures. To study working memory, behaviorally naive groups of mice were tested in the DNMTP in the T-maze according to a protocol previously described [34] with modifications to facilitate pharmacological tests [4]. Spontaneous alternation was evaluated in the Ymaze apparatus according to protocol described in detail below.

*Delayed non-match to place (DNMTP):* Behaviourally naive groups of mice were tested in the DNMTP in the T-maze according to a protocol previously described [41], with modifications to facilitate pharmacological tests. Specifically, in the present protocol, animals were first trained for 9 consecutive days of with minimal inter-trial intervals (ITI) of about 15 sec, which was necessary to attain the criterion of 90% or more of correct choices during three consecutive days (days 7-9). After this period ITI was increased semi-randomly to 180, 360, 720 and for some WT mice also 1080 seconds so that each animal was tested 6 times with each interval during four consecutive days (days 10-13 indicated). From day 13 mice were tested only twice per week beginning with training session on day one (with minimal, 15 sec ITIs) and testing pharmacological treatments on the second day. Only one pharmacological treatment was tested every 7 days. To test pro-mnemonic activities of UVI2108 and R-9CDHRA inventors used a minimal ITI, at which mice performed at chance level (50%) and which was 6min for the Rbpl-/- or Rxry-/- groups and 12 min for most WT mice with exception of two mice tested at 18min. Four mice (two wild types and one of each knockout line), were excluded from testing since their latency to choose the arm during the retention phase exceeded 3 min in more than one trial/day on three consecutive days (exclusion criterion). *Y-maze spontaneous alternation:* The Y-maze apparatus and the procedure were as previously described [41]. Briefly, each mouse was placed at the end of one arm and allowed to explore freely the apparatus for 5min, with the experimenter out of the animal's sight. Spontaneous alternation performance (SAP) was assessed visually by scoring the pattern of entries into each arm and expressed as a percentage of total number of entries, discounting the first two visits. Total entries were scored as an index of ambulatory activity in the Y-maze, but none of tested mice, had to be excluded due to low locomotor performance (score below 9 entries).

### Example 3: Analytical procedures:

High performance liquid chromatography mass spectrometry-mass spectrometry (HPLC-MS-MS) analyses were performed under dark yellow/amber light using previously validated protocol [15]. For the detection of 13,14-dihydroretinoic acid MS-MS settings were 303 -> 207 m/z using the same dwell time and collision energy comparable to MS-MS specific settings of retinoic acids. Quantification was performed as previously described [15]. For details of sample preparation see below. High performance liquid chromatography mass spectrometry (2695XE separation module; Waters) -mass spectrometry (Micromass Quattro Ultima PT; Waters) analyses were performed under dark yellow/amber light using previously validated protocol [42]. For the detection of 13,14-dihydroretinoic acid MS-MS setting were 303 -> 207 m/z using the same dwell time and collision energy comparable to MS-MS specific settings of retinoic acids. Quantification was performed comparable to RA quantification previously described in [42]. Accordingly, for sample preparation 100 mg of the material (if samples were under 100 mg, water was added up to the used standard weight: 100 mg) or 100 µl serum was diluted with a threefold volume of isopropanol, the tissues were minced by scissors, vortexed for 10 seconds, put in a ultra sonic bath for 5 minutes, shaken for 6 minutes and centrifuged at 13000 rpm in a Heraeus BIOFUGE Fresco at +4°C. After centrifugation, the supernatants were dried in an Eppendorf concentrator 5301 at 30°C. The dried extracts were resuspended with 60 µl of methanol, diluted with 40 µl of 60 mM aqueous ammonium acetate solution and transferred into the autosampler and subsequently analysed.

### Example 4: Reporter cell lines:

COS1 cells were maintained in DMEM medium with 10% FBS, 5% L-glutamine, 1% penicillin streptomycin in 24-well plates and transfections were carried out in triplicates. Cells were transfected with equal amounts of relevant plasmids including Gal-RXRα-LBD for RXR-reporter line or Gal-RARα-LBD and Gal-RXRα-LBD for RAR-RXR reporter line, a reporter plasmid (luciferase MH100-TKLuc reporter construct with GAL-binding site [35] and beta-galactosidase (for transfection efficiency calculation). For details of transfection and measurements see below. High performance liquid chromatography mass spectrometry (2695XE separation module; Waters) -mass spectrometry (Micromass Quattro Ultima PT; Waters) analyses were performed under dark yellow/amber light using previously validated protocol [2]. For the detection of 13,14-dihydroretinoic acid MS-MS setting were 303 -> 207 m/z using the same dwell time and collision energy comparable to MS-MS specific settings of retinoic acids. Quantification was performed comparable to RA quantification previously described in [2]. Accordingly, for sample preparation 100 mg of the material (if samples were under 100 mg, water was added up to the used standard weight: 100 mg) or 100 µl serum was diluted with a threefold volume of isopropanol, the tissues were minced by scissors, vortexed for 10 seconds, put in a ultra sonic bath for 5 minutes, shaken for 6 minutes and centrifuged at 13000 rpm in a Heraeus BIOFUGE Fresco at +4°C. After centrifugation, the supernatants were dried in an Eppendorf concentrator 5301at 30°C. The dried extracts were resuspended with 60 µl of methanol, diluted with 40 µl of 60 mM aqueous ammonium acetate solution and transferred into the autosampler and subsequently analysed.

COS1 cells were maintained in DMEM medium with 10% FBS, 5% L-glutamine, 1% penicillin streptomycin in 24-well plates and transfections were carried out in triplicates. Cells were transfected with equal amounts of relevant plasmids including Gal-RXRα-LBD for RXR-reporter line or Gal-RARα-LBD and Gal-RXRα-LBD for RAR-RXR reporter line, a reporter plasmid (luciferase MH100-TKLuc reporter construct with GAL-binding site [43]) and beta-galactosidase (for transfection efficiency calculation). Transfection was carried out using PEI (Sigma) as transfection reagent. DMSO solution of each ligand was added 6h later and cells were incubated for 48h at 37°C. Luciferase activity was determined in the lysates of the cells using the Luciferase Assay Kit (Promega). Measurements were made with a Wallac Victor2 multilabel counter. The signal of each sample was normalized to β-gal. activity to take the transfection efficiency and cell viability into account.

### Example 5: Binding assays:

cDNAs encoding hRXRα LBD (223-462), hRARα LBD (153-421), hRARβ LBD (169-414) and hRARγ LBD (178-423) were cloned into pET28b vector to generate N-terminal Histag fusion proteins. Purification was carried out as previously described [36,37], including a metal affinity chromatography and a gel filtration. For details of sample preparation and ESI-MS analyses see below.

cDNAs encoding hRXRaLBD (223-462), hRARaLBD (153-421), hRARβLBD (169-414) and hRARγ LBD (178-423) were cloned into pET28b vector to generate N-terminal His-tag fusion proteins. Purification was carried out as previously described [44,45], including a metal affinity chromatography and a gel filtration. Prior to ESI-MS analysis, samples were desalted on Zeba Spin desalting columns (Pierce) in 150 mM ammonium acetate (pH 8.0). ESI-MS measurements were performed on an electrospray time-of-flight mass spectrometer (MicrOTOF, Bruker Daltonics). Purity and homogeneity of the proteins were verified by mass spectrometry in denaturing conditions (samples were diluted at 2 pmol/µl in a 1:1 water-acetonitrile mixture (v/v) acidified with 1% formic acid). The mass measurements of the noncovalent complexes were performed in ammonium acetate (200 mM; pH 8.0). Samples were diluted to 8 pmol/ml in the previous buffer and continuously infused into the ESI ion source at a flow rate of 3 ml/min through a Harvard syringe pump (Harvard Apparatus model 11). A careful optimization of the interface parameters was performed to obtain the best sensitivity and spectrum quality without affecting the noncovalent complexes stability. In particular, the capillary exit (CE) ranged from 60 to 150 V with a vacuum interface pressure of 2.3 mbar and was set to 80 V. For ligand-interaction analysis, ligands were added to the proteins in a 5-fold molar excess.

*Fluorescence quenching assay* : Fluorescence spectra were measured as previously described [34,58] using a Fluoromax-4 Horiba spectrophotometer. RXRa LBD was prepared as for ESI-MS analyses and incubated with different concentrations of 9CDHRA or 9CRA in Tris 10 mM, NaCl 100 mM buffer. Quenching of tryptophan fluorescence was monitored at 10°C using 5 nm of excitation and 5 nm of emission slit-width. The excitation wavelength was 295 nm and the emission spectra were measured between 260 and 450 nm. Corrections for inner filter effect were performed and data were analyzed by Cogan plot as described [74].

### Example 6: Structure analysis of RXR-LBD in complex with (R)-9CDHRA:

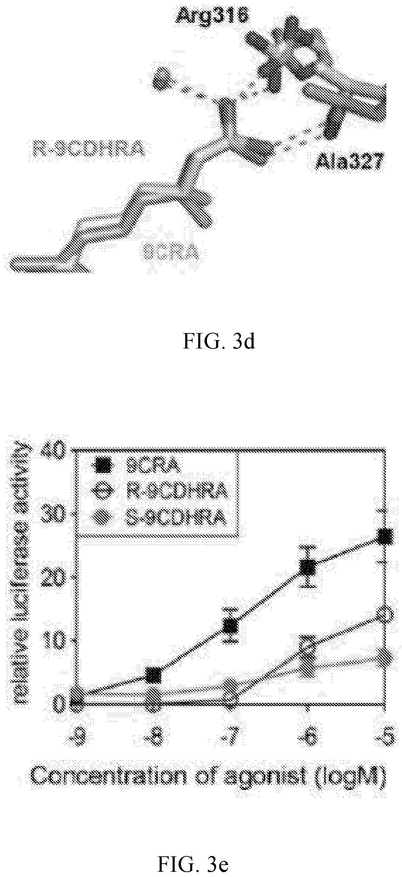

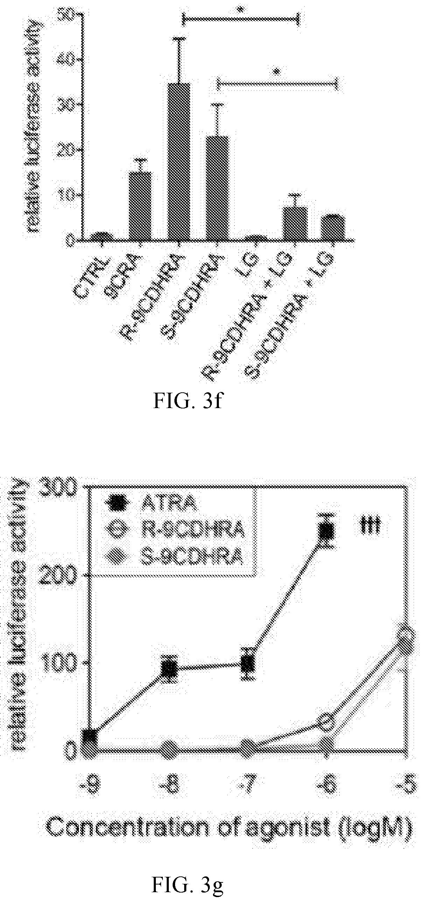

Crystals of the complex of hRXRα LBD/(*R*)-9C13,14DHRA and TIF-2 peptide were obtained at 17°C by vapor diffusion in hanging drops by mixing of 0.5 µl of the protein solution and 0.5 µl of reservoir solution which contains 50 mM calcium acetate and 18% PEG3350. The crystals were mounted in fiber loops and flash-cooled in liquid nitrogen after cryoprotection with the reservoir solution plus 5% ethylene glycol. Data collection from the frozen crystal was performed at 100 K on the beamline ID29 at the ESRF (Grenoble, France). The crystal belongs to the tetragonal space group P4₃2₁2, with one monomer per asymmetric unit. The data were integrated and scaled with HKL2000 [46] (statistics in Supporting Table 1). The structure was solved and refined as described [44]. Refinement involved iterative cycles of manual building and refinement calculations. Anisotropic scaling, a bulk solvent correction and TLS restraints were used for the refinement. Individual atomic B factors were refined isotropically. Solvent molecules were then placed according to unassigned peaks in the electron density map. For additional information see also Supporting Fig. 3 and Supporting Table 1.

**Supporting Table 1 Data collection and refinement statistics.**

| **RXRa LBD/R-DHRA /NCoA2** | |
|---|---|
| *Data processing* | |
| Resolution (Ǻ) | 25-1.8(1.86-1.80) |
| Crystal space group | P43212 |
| Cell parameters (Á) | a *= b* = 64.014; *C* = 112.066 |
| Unique reflections | 22407 |
| Mean redundancy | 7.9 (3.8) |
| *Rsym* (%)*^{a}* | 5.2 (48.1) |
| Completeness (%) | 99.2 (97.2) |

| *Refinement* | |
|---|---|
| Resolution (Ǻ) | 24-1.8 |
| Number of non-hydrogen atoms | |
| RXR-LBD | 1680 |
| Coactivator peptide | 98 |
| Ligand | 22 |
| Water molecules | 181 |
| RMSD bond length (Ǻ) | 0.006 |
| RMSD bond angles (°) | 1.065 |
| Rcryst(%)^{b} | 17.9 |
| Rfree(%)^{c} | 22.1 |
| Averaged *B* factor for non-hydrogen atoms (Ǻ²) | |
| RXR-LBD | 37.6 |
| Coactivator peptide | 44.6 |
| R-DHRA | 31.2 |
| Water | 44.5 |

| | |
|---|---|
| a Rsym = 100 x Shj\| Ihj- <Ih> \| / Shj Ihj, where ihj is the jth measurement of the intensity of reflection h and <Ih> is its mean value. b Rcryst = 100 x S∥ Fo\| - \| Fc∥ / S\| Fo\|, where \| Fo \| and \| Fc \| are the observed and calculated structure 10 factor amplitudes, respectively. c Calculated using a random set containing 10% of observations that were not included throughout refinement [Brünger A. T., (1992) The Free R Value: a Novel Statistical Quantity for Assessing the Accuracy of Crystal Structures, Nature 355, 472-474]. | |

### Example 7: Animal treatments:

(*R*)-9CDHRA, UVI2108, ATRA (Sigma), DHA (Sigma), MA (Sigma) and TTNPB (Sigma), were dissolved in ethanol and DMSO, and then mixed with sunflower oil, so that the final solution contained 3% ethanol and 3% DMSO. Vehicle treatments consisted of 3% ethanol and 3% DMSO solution in sunflower oil. Treatments were administered by intraperitoneal injections at volume/weight ratio 3 ml/kg between 8-10am and 5-6 h before the test as previously validated [4].

(*R*)-9CDHRA, UVI2108, ATRA (Sigma), DHA (Sigma), MA (Sigma) and TTNPB (Sigma), were dissolved in ethanol and DMSO, and then mixed with sunflower oil, so that the final solution contained 3% ethanol and 3% DMSO. Vehicle treatments consisted of 3% ethanol and 3% DMSO solution in sunflower oil. Treatments were administered by intraperitoneal injections at volume/weight ratio 3 ml/kg between 8-10am and 5-6 h before the test as previously validated [47].

### Example 8: Human dendritic cell (DC) generation and DNA microarray analysis

The generation and transcriptional analysis of differentiating DCs were performed as described previously [32]. Microarray data were deposited into the Gene Expression Omnibus database under accession no. GSE48573.

The generation and analysis of differentiating DCs were performed as described previously [48] with minor modifications. Briefly, human monocytes were isolated from healthy volunteer's buffy coat, obtained with a Regional Ethical Board permit from the Regional Blood Bank and isolated by Ficoll gradient centrifugation followed by immunomagnetic cell separation, and were cultured in the presence of GM-CSF and IL-4. Differentiating DCs were stimulated 18 h after plating by various agonists for 12h. The ligands were used in the following concentrations: 10 µM (*R*)-9CDHRA, 10 µM (*S*)-9CDHRA, 1 µM 9cisRA, 100 nM LG100268, 1 µM GW3965, 1 µM Rosiglitazone (RSG), 1 µM GW1516, 100 nM AM580. Experiments were performed in biological triplicates. Samples were processed and hybridized to Human Genome U133 Plus 2.0 Arrays. Data were analysed using to GeneSpring 12.6.1 software. In detail, normalization was performed using RMA summarization algorithm. Significantly regulated genes were identified using a two-fold change cut-off (only transcripts with FC>2 were included) and moderated t-test with the Benjamini-Hochberg procedure for multiple test correction (FDR<0.05). Microarray data were deposited into the Gene Expression Omnibus database under accession no. GSE48573.

### Example 9: Statistical analysis:

The comparisons of behavioral performance in Rbpl-/- and Rxry-/- mice were carried out using the protected least significant difference (PLSD) Fischer test. The pharmacological data for the treatments in WT and Rbpl-/- or Rxry-/- mice were analysed using 2-way analysis of variance (ANOVA) - with treatment and genotype as two independent factors and behavioral responses as dependent variables. The evolution of learning curves in WT, Rbpl-/- and Rxry-/- mice were done using ANOVA on repeated measures. Global and post-hoc statistical analyses were performed using student t-test for two-group comparisons or for three-group comparisons the PLSD Fischer test was used. Significant differences are indicated in the corresponding figures.

### Example 10: Chemical synthesis - synthesis of dihydroretinoids:

To confirm whether relative MS-MS signal detected at 303>207 m/z corresponds to 9CDHRA, the stereoselective synthesis of both enantiomers of 9-*cis*-13,14-dihydroretinoic acid was carried out following the previously described strategy based on a palladium-catalyzed Csp2-Csp2 Suzuki coupling [38]. Details of the stereocontrolled synthesis, purification and characterization of the (*R*)- and (*S*)-enantiomers of 9-*cis-*13,14-dihydroretinoic acid are provided below.

The preparation of (*R*)-9-cis-13,14-dihydroretinoic acid (*R*)-**1** was based on the Suzuki coupling of enantiopure trienyliodide **3** and boronic acid **2** (Scheme 1). The synthesis of **3** started with (*Z*)-stannyldienol **5** [49,50] which was transformed into the benzothiazolyl allyl sulfide **6** by Mitsunobu reaction with the corresponding thiol and subsequently oxidized to sulfone **7** with H₂O₂ and a peroxymolybdate (VI) reagent [41] at -10 °C. The Julia-Kocienski olefination [12,13] was performed using a slight excess of base (NaHMDS, 1.15 equiv.) and 1.7 equivalents of enantiopure aldehyde (*R*)-**8**.[14,15]. As anticipated from previous findings on the stereochemical outcome of the reactions of allylsulfones and aldehydes[56,57], the newly formed olefin of trienyl ester (*R*)-**9** is of *Z*-geometry (which was confirmed by NOE experiments). Treatment of the precursor stannane with a solution of iodine in CH₂Cl₂ produced the iodide (*R*)-**3** via Sn-I exchange and iodine-promoted isomerization of the 9*Z*,11*Z* diene to the desired 9*Z*,11*E* geometric isomer (as confirmed by NOE experiments).

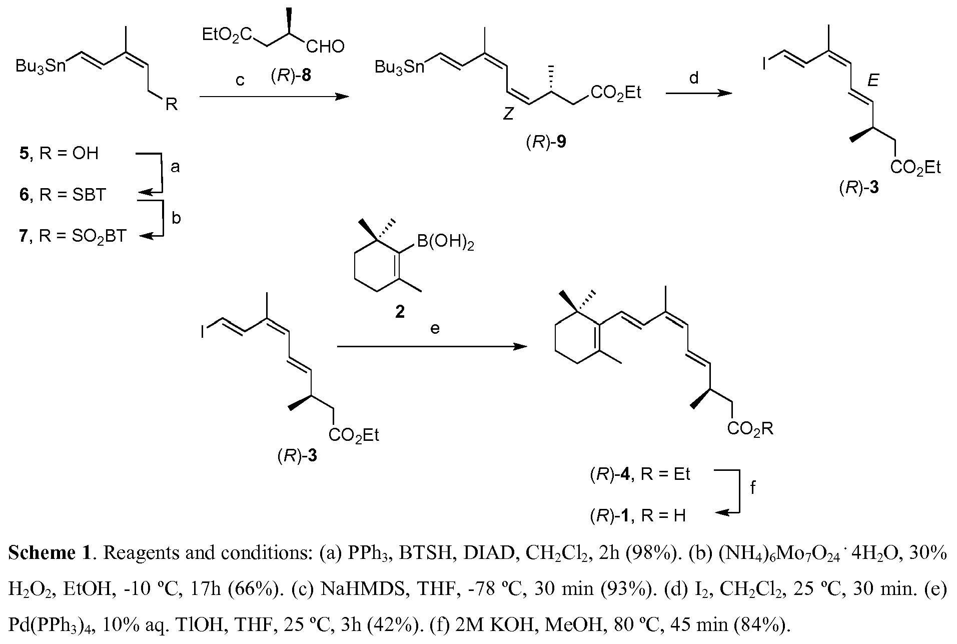

The Suzuki reaction of freshly prepared boronic acid **2** and dienyl iodide (*R*)-**3** using Pd(PPh₃)₄ as catalyst and 10% aq. TlOH as base in THF at ambient temperature, followed by immediate work-up afforded ethyl (*R*)-9-*cis*-13,14-dihydroretinoate (*R*)-**4** in 78% yield. Saponification of (*R*)-**4** provided in 84% yield the desired carboxylic acid (*R*)-**1** without detectable loss of stereochemical integrity.

Following the general scheme, enantiomer (*S*)-**1** was also prepared with similar efficiency.

### Preparation and Characterization of Compounds - General procedures

Solvents were dried according to published methods and distilled before use. All other reagents were commercial compounds of the highest purity available. Unless otherwise indicated all reactions were carried out under argon atmosphere, and those not involving aqueous reagents were carried out in oven-dried glassware. Analytical thin layer chromatography (TLC) was performed on aluminum plates with Merck Kieselgel 60F254 and visualized by UV irradiation (254 nm) or by staining with an ethanolic solution of phosphomolibdic acid. Flash column chromatography was carried out using Merck Kieselgel 60 (230-400 mesh) under pressure. Electron impact (EI) mass spectra were obtained on a Hewlett-Packard HP59970 instrument operating at 70 eV. Alternatively an APEX III FT-ICR MS (Bruker Daltonics), equipped with a 7T actively shielded magnet was used and ions were generated using an Apollo API electrospray ionization (ESI) source, with a voltage between 1800 and 2200 V (to optimize ionization efficiency) applied to the needle, and a counter voltage of 450 V applied to the capillary. For ESI spectra samples were prepared by adding a spray solution of 70:29.9:0.1 (v/v/v) CH₃OH/water/formic acid to a solution of the sample at a v/v ratio of 1 to 5% to give the best signal-to-noise ratio. High Resolution mass spectra were taken on a VG Autospec instrument. ¹H NMR spectra were recorded in C₆D₆ and acetone-d₆ at ambient temperature on a Bruker AMX-400 spectrometer at 400 MHz with residual protic solvent as the internal reference.

### (2Z,4E)-1-(Benzothiazol-2-yl)sulfanyl-5-(tri-n-butylstannyl)-3-methylpenta-2,4-diene (6).

A solution of (2*Z*,4*E*)-3-methyl-5-(tributylstannyl)penta-2,4-dien-1-ol (1.0 g, 2.58 mmol), 2-mercaptobenzothiazol (0.65 g, 3.87 mmol) and PPh₃ (1.10 g, 4.21 mmol) in THF (14 mL) was stirred for 5 min at 0 °C. A solution of DIAD (0.77 mL, 3.87 mmol) in THF (5 mL) was added dropwise and the mixture was stirred for 30 min at 25 °C. The solvent was removed and the residue was purified by column chromatography (C18-silicagel, CH₃CN) to afford 1.11 g (78%) of a colorless oil identified as (2*Z*,4*E*)-1-(Benzothiazol-2-yl)sulfanyl-5-(tri-*n*-butylstannyl)-3-methylpenta-2,4-diene **6. ¹H NMR** (400 MHz, C₆D₆) δ 8.04 (d, *J* = 8.2 Hz, 1H, CH), 7.46 (d, *J=* 19.2 Hz, ³*J*_{SnH} = 71.2 Hz, 1H, CH), 7.34 (d, *J=* 8.0 Hz, 1H, CH), 7.21 (ddd, *J=* 8.3, 7.3, 1.2 Hz, 1H, CH), 7.01 (ddd, *J* = 8.3, 7.4, 1.1 Hz, 1H, CH), 6.66 (d, *J* = 19.2 Hz, ²*J*_{SnH} = 71.2 Hz, 1H, CH), 5.65 (t, *J* = 8.1 Hz, 1H, CH), 4.37 (d, *J* = 8.2 Hz, 2H, CH₂), 1.86 (s, 3H, CH₃), 1.8-1.6 (m, 6H, CH₂), 1.5-1.4 (m, 6H, CH₂), 1.1-1.0 (m, 15H, CH₃ + CH₂) ppm. **¹³C NMR** (100 MHz, C₆D₆) δ 166.4 (s), 153.7 (s), 142.6 (d), 138.6 (s), 135.7 (s), 132.0 (d), 125.9 (d), 124.1 (d), 122.1 (d), 121.7 (d), 121.0 (d), 30.2 (t), 29.3 (t, 3x), 27.6 (t, 3x), 19.8 (q), 13.8 (q, 3x), 9.7 (t, 3x) ppm; **IR** (NaCl) v 2955 (s, C-H), 2923 (s, C-H), 2870 (m, C-H), 2850 (m, C-H), 1460 (s), 1427 (s) cm⁻¹. **HRMS** (ESI⁺) *m*/*z* calcd for C₂₅H₄₀NS₂¹²⁰Sn: 538.1620; found: 538.1621. **(2*Z*,4*E*)-1-(Benzothiazol-2-yl)sulfonyl-5-(tri-*n*-butylstannyl)-3-methylpenta-2,4-diene (7).**

To a solution of (2*Z*,4*E*)-1-(benzothiazol-2-yl)sulfanyl-5-(tri-*n*-butylstannyl)-3-methylpenta-2,4-diene 6 (0.48 g, 0.89 mmol) in EtOH (9 mL), at -10 °C, was added a solution of (NH₄)₆Mo₇O₂₄·4H₂O, (0.44 g, 0.36 mmol) in aqueous hydrogen peroxide (35%, 7.7 mL, 89.1 mmol). After stirring for 17 h at -10 °C, the mixture was quenched with H₂O and extracted with Et₂O (3x). The combined organic layers were washed with brine (3x) and dried (Na₂SO₄), and the solvent was removed. The residue was purified by chromatography (C18-silicagel, MeOH) to afford 0.33 g (66%) of a colorless oil identified as (2*Z*,4*E*)-1-(benzothiazol-2-yl)sulfonyl-5-(tri-*n*-butylstannyl)-3-methylpenta-2,4-diene 7. **¹H NMR** (400 MHz, C₆D₆) δ 8.10 (d, *J=* 8.2 Hz, 1H, CH), 7.18 (d, *J* = 19.2 Hz, 1H, CH), 7.16 (t, *J* = 7.5 Hz, 1H, CH), 7.12 (t, *J* = 8.1 Hz, 1H, CH), 6.98 (t, *J* = 7.7 Hz, 1H, CH), 6.59 (d, *J* = 19.2 Hz, ²*J*_{SnH} = 68.2 Hz, 1H, CH), 5.43 (t, *J* = 8.0 Hz, 1H, CH), 4.32 (d, *J* = 8.1 Hz, 2H, CH₂), 1.8-1.6 (m, 9H, CH₃ + CH₂), 1.6-1.4 (m, 6H, CH₂), 1.1-1.0 (m, 15H, CH₃ + CH₂) ppm. **¹³C NMR** (100 MHz, C₆D₆) δ 167.1 (s), 152.9 (s), 143.1 (s), 141.7 (d), 136.9 (s), 134.4 (d), 127.3 (d), 127.1 (d), 125.0 (d), 122.1 (d), 112.0 (d), 53.3 (t), 29.3 (t, 3x), 27.5 (t, 3x), 20.0 (q), 13.8 (q, 3x), 9.6 (t, 3x) ppm; **IR** (NaCl) v 2955 (s, C-H), 2923 (s, C-H), 2850 (m, C-H), 1467 (m), 1333 (s), 1151 (s) cm⁻¹. **HRMS** (ESI⁺) *m*/*z* calcd for C₂₅H₃₉NNaO₂S₂¹²⁰Sn: 592.1338; found: 592.1334. UV (MeOH) *λ*ₘₐₓ 239 nm.

### (3S,4Z,6Z,8E)-Ethyl 3,7-Dimethyl-9-(tri-n-butylstannyl)nona-4,6,8-trienoate ((S)-9).

A cooled (-78 °C) solution of (2*Z*,4*E*)-1-(benzothiazol-2-yl)sulfonyl-5-(tri-*n*-butylstannyl)-3-methylpenta-2,4-diene (0.115 g, 0.20 mmol) in THF (9 mL) was treated with NaHMDS (0.23 mL, 1M in THF, 0.23 mmol). After stirring for 30 min at this temperature, a solution of (*S*)-ethyl 3-methyl-4-oxobutanoate (0.044 g, 0.30 mmol) in THF (4.5 mL) was added and the resulting mixture was stirred for 1h at -78 °C and 3h letting the temperature reach to rt. Et₂O and water were added at low temperature and the mixture was warmed up to room temperature. It was then diluted with Et₂O and the layers were separated. The aqueous layer was extracted with Et₂O (3x), the combined organic layers were dried (Na₂SO₄) and the solvent was removed. The residue was purified by column chromatography (C-18 silica gel, MeOH) to afford 0.94 g (93%) of a pale yellow oil identified as (3*S*,4*Z*,6*Z*,8*E*)-ethyl 3,7-dimethyl-9-(tri-*n*-butylstannyl)nona-4,6,8-trienoate (*S*)-**9**. **[α]²⁸_{D}** +11.5° (*c* 1.22, MeOH). **¹H NMR** (400 MHz, C₆D₆) δ 7.60 (d, *J=* 19.1 Hz, ³*J*_{SnH} = 65.1 Hz, 1H, CH), 6.85 (t, *J* = 11.4 Hz, 1H, CH), 6.64 (d, *J* = 19.2 Hz, ²*J*_{SnH} = 71.9 Hz 1H, CH), 6.58 (d, *J* = 11.9 Hz, 1H, CH), 5.31 (t, *J* = 10.4 Hz, 1H, CH), 4.03 (q, *J* = 7.1 Hz, 2H, CH₂), 3.5-3.3 (m, 1H, CH), 2.4-2.2 (m, 2H, CH₂), 2.02 (s, 3H, CH₃), 1.8-1.6 (m, 6H, CH₂), 1.6-1.4 (m, 6H, CH₂), 1.2-0.9 (m, 18H, CH₃ + CH₂) ppm. **¹³C NMR** (100 MHz, C₆D₆) δ 171.3 (s), 143.2(d), 135.8 (d), 135.4 (s), 130.7 (d), 123.8 (d), 122.9 (d), 59.8 (t), 41.8 (t), 29.3 (t, 3x), 29.2 (d), 27.5 (t, 3x), 20.7 (q), 20.3 (q), 14.0 (q), 13.7 (q, 3x), 9.6 (t, 3x) ppm. **IR** (NaCl) v 2957 (s, C-H), 2924 (s, C-H), 2871 (m, C-H), 2851 (m, C-H), 1737 (s, C=O), 1459 (m), 1160 (m) cm⁻¹. **HRMS** (ESI⁺) *m*/*z* calcd for C₂₅H₄₆NaO₂¹²⁰Sn: 521.2416; found: 521.2408. **UV** (MeOH) *λ*ₘₐₓ 285 nm.

**(3*R*,4*Z*,6*Z*,8*E*)-Ethyl 3,7-Dimethyl-9-(tri-*n*-butylstannyl)nona-4,6,8-trienoate ((*R*)-9). [α]²⁹_{D}** -10.3° (c 1.25, MeOH).

### (3R,4E,6Z,8E)-Ethyl 9-Iodo-3,7-dimethylnona-4,6,8-trienoate ((R)-3).

To a solution of (3*R*,4*Z*,6*Z*,8*E*)-ethyl 3,7-dimethyl-9-(tri-*n*-butylstannyl)nona-4,6,8-trienoate ***(R)-9*** (0.060 g, 0.121 mmol) in CH₂Cl₂ (5.3 mL) was added dropwise iodine (0.046 g, 0.182 mmol) in CH₂Cl₂ (2.8 mL) and the resulting mixture was stirred for 30 min at 25 °C. A saturated Na₂S₂O₃ solution was added and the reaction mixture was extracted with Et₂O (3x), the combined organic layers were dried (Na₂SO₄) and the solvent was removed. The residue was purified by column chromatography (silica gel, 97:3 hexane/Et₃N) to afford 0.037 g (92%) of a pale yellow oil identified as (3*R*,4*E*,6*Z*,8*E*)-ethyl 9-iodo-3,7-dimethylnona-4,6,8-trienoate (*R*)-**3. [α]²⁴_{D}** +14.8° (c 0.74, MeOH). **¹H NMR** (400 MHz, C₆D₆) δ 7.65 (d, *J* = 14.5 Hz, 1H, CH), 6.28 (dd, *J* = 14.9, 11.3 Hz, 1H, CH), 6.05 (d, *J* = 14.5 Hz, 1H, CH), 5.64 (d, *J* = 11.1 Hz, 1H, CH), 5.44 (dd, *J* = 15.0, 7.8 Hz, 1H, CH), 3.95 (q, *J* = 7.1 Hz, 2H, CH₂), 2.64 (dt, *J* = 14.1, 7.0 Hz, 1H, CH), 2.15 (dd, *J* = 14.9, 7.1 Hz, 1H, CH), 2.06 (dd, *J* = 14.9, 7.3 Hz, 1H, CH), 0.97 (t, *J* = 7.2 Hz, 3H, CH₃), 0.88 (d, *J* = 6.8 Hz, 3H, CH₃) ppm. **¹³C NMR** (100 MHz, C₆D₆) δ 171.9 (s), 142.7 (d), 140.4 (d), 132.7 (s), 130.9 (d), 124.6 (d), 78.5 (d), 60.5 (t), 41.8 (t), 34.5 (d), 20.4 (q), 19.9 (q), 14.7 (q) ppm. **IR** (NaCl) v 2972 (m, C-H), 2932 (m, C-H), 1731 (s, C=O), 1666 (m), 1180 (m) cm⁻¹. **HRMS** (ESI⁺) *m*/*z* calcd for C₁₃H₁₉INaO₂: 357.0322; found: 357.0316. **UV** (MeOH) *λ*ₘₐₓ 275 nm.

**(3*S*,4*E*,6*Z*,8*E*)-Ethyl 9-Iodo-3,7-dimethylnona-4,6,8-trienoate ((*S*)-3). [α]²³_{D}** -16.7° (c 0.72, MeOH).

**(9*Z*,13*R*)-Ethyl 13,14-Dihydroretinoate ((*R*)-4).** To a solution of (3*R*,4*E*,6*Z*,8*E*)-ethyl 9-iodo-3,7-dimethylnona-4,6,8-trienoate *(R)*-**3** (0.036 g, 0.107 mmol) in THF (2.3 mL) was added Pd(PPh₃)₄ (0.013 g, 0.011 mmol). After 5 min at room temperature, 2,6,6-trimethylcyclohex-1-enylboronic acid **2** (0.027 g, 0.161 mmol) was added in one portion followed by TlOH (10% aqueous solution, 0.75 mL, 0.407 mmol). After stirring for 3 h at 25 °C, Et₂O was added and the reaction mixture was filtered through a short pad of Celite®. The filtrate was washed with NaHCO₃ (sat) and the organic layer was dried (Na₂SO₄) and the solvent was removed. The residue was purified by column chromatography (silica gel, 97:3 hexane/Et₃N) to afford 0.028 g (78%) of a pale yellow oil identified as (9*Z*,13*R*)-ethyl 13,14-dihydroretinoate (*R*)-**4**. **[α]²³_{D}** +14.2° (*c* 0.48, MeOH). **¹H NMR** (400 MHz, C₆D₆) δ 6.90 (d, *J* = 16.0 Hz, 1H, CH), 6.71 (dd, *J* = 14.9, 11.2 Hz, 1H, CH), 6.28 (d, *J* = 16.0 Hz, 1H, CH), 5.96 (d, *J* = 11.1 Hz, 1H, CH), 5.51 (dd, *J* = 15.0, 7.8 Hz, 1H, CH), 3.94 (q, *J* = 7.2 Hz, 2H, CH₂), 2.77 (dt, *J* = 14.1, 7.1 Hz, 1H, CH), 2.21 (dd, *J* = 14.8, 7.3 Hz, 1H, CH), 2.11 (dd, *J* = 14.8, 7.2 Hz, 1H, CH), 1.95 (t, *J* = 6.1 Hz, 2H, CH₂), 1.90 (s, 3H), 1.79 (s, 3H), 1.66 - 1.52 (m, 2H), 1.52 - 1.41 (m, 2H), 1.11 (s, 6H), 0.95 (t, *J* = 7.1 Hz, 3H, CH₃), 0.94 (d, *J* = 6.8 Hz, 3H, CH₃) ppm. **¹³C NMR** (100 MHz, C₆D₆) □ 171.4 (s), 138.3 (s), 137.6 (d), 132.7 (s), 130.6 (d), 129.1 (d), 129.0 (s), 127.9 (d), 124.8 (d), 59.8 (t), 41.6 (t), 39.6 (t), 34.2 (s), 34.1 (d), 33.0 (t), 28.9 (q, 2x), 21.8 (q), 20.4 (q), 20.1 (q), 19.5 (t), 14.1 (q) ppm. **IR** (NaCl) v 2961 (s, C-H), 2929 (s, C-H), 2866 (m, C-H), 1737 (s, C=O), 1455 (m), 1372 (m), 1167 (m) cm⁻¹. **HRMS** (ESI⁺) *m*/*z* calcd for C₂₂H₃₅O₂: 331.2632; found: 331.2625. **UV** (MeOH) λₘₐₓ 287 nm (ε = 20000 L·mol⁻¹·cm⁻¹).

**(9*Z*,13*S*)-Ethyl 13,14-dihydroretinoate ((*S*)-4). [α]²²_{D}** -15.5° (*c* 0.51, MeOH).

**(9*Z*,13*R*)-13,14-Dihydroretinoic Acid ((*R*)-1).** To a solution of (9*Z*,13*R*)-ethyl 13,14-dihydroretinoate (*R*)-**4** (0.023 g, 0.069 mmol) in MeOH (4.7 mL) was added KOH (2M aqueous solution, 1.1 mL, 2.27 mmol) and the reaction mixture was stirred for 45 min at 80 °C. After letting the reaction cool down to room temperature, CH₂Cl₂ and brine were added and the layers were separated. The aqueous layer was washed with H₂O (3x). The combined aqueous layers were acidified with HCl 10% and extracted with CH₂Cl₂ (3x). The combined organic layers were dried (Na₂SO₄) and the solvent was removed. The residue was purified by column chromatography (silica gel, gradient from 95:5 to 90:10 CH₂Cl₂/MeOH) to afford 0.017 g (84%) of a pale yellow oil identified as (9*Z*,13*R*)-13,14-dihydroretinoic acid (*R*)-**1**. **[α]²²_{D}** +7.1° (*c* 0.67, MeOH). **¹H NMR** (400 MHz, acetone-d₆): δ= 6.66 (d, *J* = 16.0 Hz, 1H), 6.60 (dd, *J* = 15.0, 11.2 Hz, 1H), 6.18 (d, *J* = 16.0 Hz, 1H), 5.93 (d, *J* = 11.1 Hz, 1H), 5.65 (dd, *J* = 15.0, 7.5 Hz, 1H), 2.73 (dt, *J* = 13.9, 7.0 Hz, 1H), 2.4 - 2.2 (m, 2H), 2.1 - 2.0 (m, 1H), 1.91 (s, 3H), 1.72 (s, 3H), 1.7 - 1.6 (m, 2H), 1.5 - 1.4 (m, 2H), 1.08 (d, *J* = 6.8 Hz, 3H), 1.03 (s, 6H) ppm. **¹³C NMR** (100 MHz, acetone-d₆) □ 173.5 (s), 138.9 (s), 138.8 (d), 133.4 (s), 131.1 (d), 129.8 (s), 129.7 (d), 128.5 (d), 125.4 (d), 41.8 (t), 40.3 (t), 34.9 (s), 34.6 (d), 33.6 (t), 29.4 (q), 22.1 (q), 20.7 (q), 20.6 (q), 20.0 (t) ppm. **IR** (NaCl) v 2957 (s, C-H), 2923 (s, C-H), 2855 (m, C-H), 1709 (s, C=O), 1446 (m), 1290 (m) cm⁻¹. **HRMS** (ESI⁺) *m*/*z* calcd for C₂₀H₃₁O₂: 303.2319; found: 303.2313. **UV** (MeOH) λₘₐₓ 289 nm (ε = 17600 L·mol⁻¹·cm⁻¹).

**(9*Z*,13*S*)-13,14-Dihydroretinoic Acid ((*S*)-1). [α]²⁴_{D}** -6.9° (*c* 0.26, MeOH).

### Example 11 : Experimental procedure Affective and memory deficits in Rbpl-/- mice:

### Animals:

Rbpl-/- and Rxrγ-/- mutants as well as their wild type (WT) control mice were raised on a mixed genetic background (60% C57BL/6J and 40% 129SvEms/j) from heterozygous crosses as described [58; 33], and tested at the age of 3-6 months. All mice were housed in groups of 4-5 mice per cage in a 7am-7pm light/dark cycle in individually ventilated cages (Techniplast, Italy). Food and water were freely available. All experiments were carried out in accordance with the European Community Council Directives of 24 November 1986 (86/609/EEC) and in compliance with the guidelines of CNRS and the French Agricultural and Forestry Ministry (decree 87848).

For social defeat stress protocol we used C57BL/6N mice which were transferred from Taconic (France) at the age of 6 weeks and housed in groups of 4 per cage. After 1 week of habituation period they were subjected to the social defeat stress. CD1 purchased from Charles River (France), were used as aggressors in this test.

*Behavioural procedures:* All behavioural tests were carried out in the Institute Clinique de la Souris (http://www.ics-mci.fr/) according to standard operating procedures.

### Forced swim test:

The forced swim paradigm [77] was carried out between 1pm and 4pm in a 2-litre glass beaker half-filled with water at 22-23°C (the water depth was 17 cm). All mice were tested only once in this task. To this end, each mouse was lowered gently into the water and the time of immobility was scored during a 6-minute testing period. The mouse was judged immobile when it floated in an upright position and made only small movements to keep its head above the water. After 6 min, the mouse was taken out of the water, left to dry under a red light lamp and returned to its home cage. The immobility scores of each animal were used as an index of despair behaviour.

*Sucrose preference test:* This task, designed to measure hedonic behaviours in mice [76], is based on the palatable nature of sucrose observed in a number of mouse strains. On the first day of the test, sucrose-naive mice were placed in individual cages at 11am and left there with water and food for habituation period. At 5pm one water bottle was replaced with two bottles: one containing water and another 0.8% sucrose solution. Three hours later (8pm) the bottles were weighed to measure liquid consumption and were replaced in cages until morning. The measures of an overnight consumption were then carried out for additional day to evaluate sucrose preference. Mice were not water deprived at any moment, in order to measure spontaneous sucrose preference and exclude any potential emotional confounds induced by stress of water deprivation. The sucrose preference was expressed as the percent of sucrose solution consumed with respect to total liquid consumption.

*Social defeat stress:* Social defeat stress procedure was a modified version of the protocol previously described [75]. C57BL/6N mice were defeated chronically for 10 consecutive days. Every day they were exposed to the physical contact with an unfamiliar CD1 aggressor in its home cage for maximal interaction time of 5 minutes. After each session of physical stress, C57BL/6N and CD1 mice were separated by a perforated wall and maintained in sensory contact for 24h. After the last session of stress, mice were transferred into new cages and housed separately throughout the behavioral tests period. C57BL/6N control mice, similarly to the experimental animals, were housed two per cage separated by a metal perforated wall. Every day, they were exposed to the physical contact for 5 minutes.

Animals were then tested in the forced swim and sucrose tests.

### Example 12: Affective and memory deficits in Rbp1-/- mice are associated with deficient RXR-signaling

*Rbp1-*/*-* mice displayed depressive-like behavioural deficits including increased despair illustrated by long immobility states in the forced swim test (Fig. 5a) and anhedonia on evidence of reduced sucrose preference (Supporting Fig. 1a). Such deficits resemble phenotype of mice carrying null mutation for RXRγ (Fig. 5a, Supporting Fig. 1a and [59] suggesting that RXR signaling is compromised in Rbpl-/- mice. To challenge this hypothesis functionally, we took advantage of the sensitivity of the forced swim test to reveal antidepressant activities of acute treatments with RXR-agonists [73]. All*-trans* retinoic acid (ATRA) which in vivo can rapidly be transformed to the RXR agonist, 9CRA, similarly to a synthetic RXR agonist, UVI2108 (known also as BMS649) reduced significantly immobility time of Rbpl-/- mice in the forced swim test (Fig. 5b). Distinct RXR agonists of synthetic (methoprene acid) or nutritional origin (DHA) also normalized increased despair of Rbpl-/- mice and such effect was independent of RAR activation since TTNPB, a synthetic RAR agonist did not display such effects (Supporting Fig. 1b). RXRγ appeared as the functionally predominant RXR in mediating such activities in the forced swim as Rxr γ-/- mice displayed increased despair behaviours, which were not normalised by RXR agonists (Fig. 5b and Supporting Fig. 1b).

### Example 13: R-9CDHRA supplementation reverses behavioural changes in Rbpl-/- mice

In order to address relevance of 9CDHRA in modulation of RXR functions *in vivo* inventors tested whether R-9CDHRA can reverse behavioural deficits in Rbpl-/- mice. Acute treatment with R-9CDHRA reduced in dose dependent manner immobility of knockout mice in the forced swim test attaining maximal effect already at 1 mg/kg, which was comparable to antidespair effect of synthetic RXR agonist UVI2108 at the same dose or 5 mg/kg treatment with ATRA (compare Figs. 5 and 1b). Effects of treatments were not evident in WT mice, which may be related to the low baseline immobility in this strain and in consequence floor effect. Such activities were mediated by RXRγ as 2 mg treatment with R-9CDHRA did not improve performance of Rxry-/- mice, which remained immobile for 120 ± 25 sec as compared to 119 ± 19 sec in vehicle treated Rxry-/- animals.

Similarly to antidespair effects in the forced swim test, 1 or 2 mg/kg of R-9CDHRA also improved performance of Rbpl-/- mice in memory tests. Such treatments increased the rate of successful choices of Rbp1-/-, which performed significantly better than 50% of chance level when tested at inter-trial interval of 6 min in DNMTP test (Fig. 3h). Treatment with 2 mg/kg of R-9CDHRA raised also performance of WT mice to about 70% of correct choices when tested at long inter-trial intervals of 12 or 18 min at which the same WT mice performed at chance level (50% of correct choices) if treated with vehicle. Such treatment did not improve performance of Rxrγ-/- mice which performed at 57 ± 7% of correct choices, providing further evidence that compromised RXRγ function associated with reduced levels of 9CDHRA is at the origin of the deficits observed in Rbpl-/- animals.

### R-9CDHRA supplementation prevents depressive behaviours in chronic stress model of depression.

Stress is an important environmental factor in the etiology of depression. To test efficiency of 9cDHRA for treatment of depressive behaviours induced by stress, we used social defeat stress animal model [75][78]. Ten days of short daily physical contacts with resident dominant CD1 male followed by subsequent sensory contact efficiently induced despair in the forced swim task (fig. 7a). Treatment with 1 or 3mg/kg of 9cDHRA during the stress protocol efficiently normalised immobility time which was comparable with control non-stressed mice. In contrast to the lower dose, treatment with 3mg/kg of 9cDHRA displayed anti-despair effects as it was significantly lower than immobility time observed in stressed, non- treated mice indicating thus that 9cDHRA displays dose effect in controlling this behavioural parameter. The effect of 9cDHRA was comparable with activity of synthetic panRXR agonist UVI2108, suggesting critical role of RXR activation in attaining anti-despair activity of by 9cDHRA. In addition to despair behaviors chronic stress induced also anhedonia reflected by absence of preference of sweetened drink which was consumed at the level not significantly different from 50% reflecting random choice (fig. 7b). Anhedonia was abolished in stressed mice treated already with low dose of 9cDHRA (1mg/kg) as illustrated by sucrose preference significantly exceeding a chance level of 50%, although this preference was even more marked after treatment with higher dose of 3mg/kg of 9cDHRA. A panRXR agonist UVI2108 displayed activities similar to 9cDHRA supporting the involvement of RXR activation by 9cDHRA in antidepressant activities.

### Discussion:

In order to search for endogenous retinoids that may act as RXR ligand(s), the inventors first employed behavioral and pharmacological analyses sensitive to RXR signaling as a tool to identify animal models with reduced RXR signaling. In particular, inventors focused on spatial working memory previously reported as dependent on RXR and not RAR functions, including ligand-dependent RXR activities [4]. Using delayed non-match to place (DNMTP) task, inventors found that mice carrying a null mutation of cellular retinol binding protein I (RBP1), known for its role in retinoid metabolism [24], display memory deficits which phenocopy the effect of the loss of function of Rxrγ, a functionally predominant RXR in control of working memory (Fig. 1a and ref. [4]). In particular, *Rbp1-*/*-* and *Rxrγ* -/- mice performed significantly worse when compared to wild type (WT) mice at 3 or 6 min inter-trial intervals (ITI) in DNMTP task, attaining chance level (complete forgetting) already at 6 min, whereas WT mice performed at chance level only at 12 or 18 min depending on individual (Fig. 1a, see grey part of the left panel). These data suggest that RXR signaling is compromised in *Rbp1-*/*-* mice.

To challenge this hypothesis functionally, inventors took advantage of the sensitivity of working memory performance in delayed task to treatments with RXR agonists [4]. Activation of RXR signaling by the synthetic RXR agonist, UVI2108 (also known as SR11217 or BMS649) or by ATRA, which under pharmacological conditions is rapidly transformed *in vivo* to RXR agonist 9CRA [8,25], reversed memory deficits in *Rbp1-*/*-* mice, but remained ineffective in mice lacking RXRγ (Fig. 1b). Working memory deficits were also observed in a distinct, rodent specific memory test of spontaneous alternation in the Y-maze (Supporting Fig. S1c). Treatments with ATRA, UVI2108 and other RXR agonists, including DHA and methoprene acid, but not pan-RAR agonist TTNPB led to pro-mnemonic effects in *Rbp1-*/*-,* but not *Rxrγ* -/mice (Supporting Fig. S1c), supporting the possibility of compromised RXR signaling due to reduced availability of RXR ligand(s) in *Rbp1-*/*-* mice. Accordingly, reduced expression of RXRγ could not explain behavioral deficits in *Rbp1-*/*-* mice. On the contrary, expression of RXRγ was clearly increased in *Rbp1-*/-striatum attaining level of 3.2 ± 0.6 in *Rbp1-*/*-* mice as compared to 1.2 ± 0.3 arbitrary RNA units (qRT-PCR).

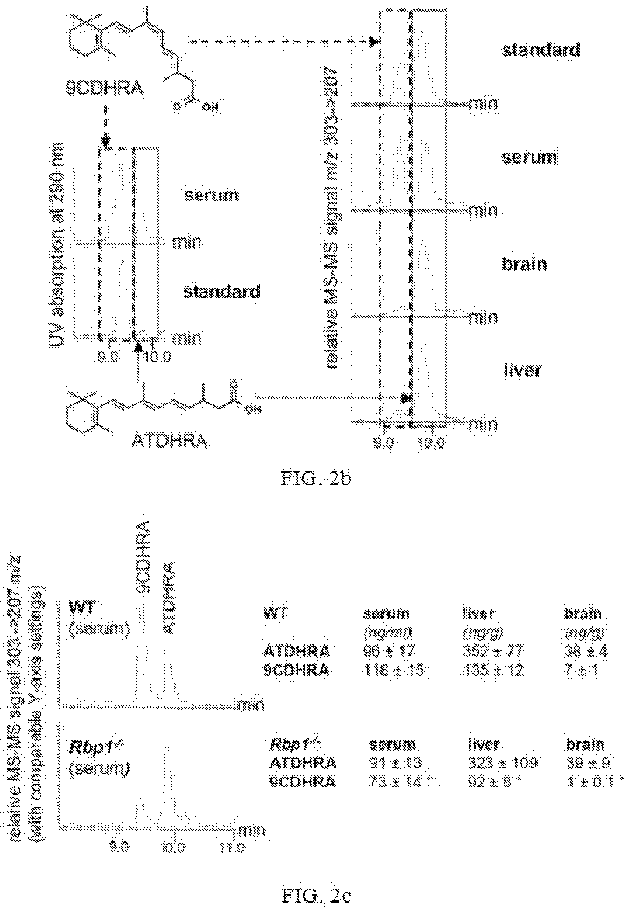

To evaluate RXR ligand availability in *Rbp1*^{*-*/*-*} mice, inventors first addressed concentration of 9CRA in mouse brain and serum. Using a sensitive method of retinoic acid detection based on HPLC separation followed by highly specific DAD detection and destructive MS-MS [15], inventors clearly identified ATRA in serum (0.3 ± 0.1 ng/ml) and brain (0.6 ± 0.1 ng/g) samples from WT mice, whereas in the range of 9CRA elution no conclusive peak was identified indicating that 9CRA levels were under our detection limit of 0.1 ng/g and thereby too low for RXR-activation in WT (Fig. 2a) and *Rbp1*^{*-*/*-*} animals. Inventors then focused on dihydroretinoids described as novel endogenous retinoids [26,27]. Using stereo- and enantio-controlled organic synthesis approaches we obtained a series of dihydroretinoids, including all-*trans*-13,14-dihydroretinoic acid (ATDHRA) and its stereoisomer 9-*cis*-13,14-dihydroretinoic acid (9CDHRA; see Materials and methods for details of its synthesis), which inventors next used as reference molecules in HPLC-MS-MS analyses. Focusing such analyses on liver, as major site of Rbp1 expression, serum through which retinoids are distributed to target organs and brain, with discrete areas expressing Rbp1 [28], inventors identified two major peaks, which co-eluted with standards of ATDHRA and 9CDHRA at UV specific absorption of 290 nm (Fig. 2b, left panel). Such co-elution was also observed at dihydroretinoid-specific MS-MS settings (Fig. 2b, right panel). Concentrations of 9CDHRA were high in serum samples attaining 118 ± 15 ng/ml (corresponding to ∼4 x 10⁻⁷M), 135 ± 12 ng/g in mouse liver (corresponding to a concentration of ∼7x10⁻⁷M) and relatively low (7 ± 1 ng/g, corresponding to ∼2 x 10⁻⁸M) in brain. A direct comparison of these retinol metabolites in WT and littermate *Rbp1*^{*-*/*-*} mice (Fig. 2c) showed comparable concentration of ATDHRA in contrast to significantly decreased 9CDHRA levels in serum, liver and brain of *Rbp1*^{*-*/*-*} mice. Importantly, whereas such decrease in serum may be at the origin of systemic reduction of RXR signaling, almost complete loss of 9CDHRA availability in *Rbp1*^{*-*/*-*} brain suggest more significant reduction of local RXR signalling in this organ. Furthermore, whole brain measures reflect most probably more dramatic changes of 9CDHRA levels in discrete brain areas expressing Rbp1 [28]. Unfortunately it is technically impossible to identify retinoid concentrations in these small areas, which can be only prompted by whole brain measures.

Direct evidence for 9CDHRA binding to RXR was given by electrospray ionisation mass spectrometry (ESI-MS) performed in non-denaturing conditions with purified RXR ligand binding domains (LBD). In order to evaluate relative affinities of R- and S-enantiomers and 9CDHRA for RXR LBD, titration experiments were monitored by ESI-MS. As shown in Fig. 3a and b, all retinoids bind to hRXRα LBD used in these studies as model RXR LBD due to high conservation of LBD structure among all RXRs. Analyses of peak amplitude revealed that R-9CDHRA has approximately 30% lower affinity than 9CRA, but about 65% higher affinity than S-9CDHRA. Quantitative binding affinities to RXRa LBD obtained by fluorescence quenching assay (Supporting Fig. S2) are equal to 90 ± 20 nM for R-9CDHRA and 20 ± 10 nM for 9CRA, and fall in the range of published Kd [74] indicating that 9CDHRA binds RXRs with high affinity at concentrations which are physiologically relevant. Since 9CRA can bind to RARs we also tested affinity of 9CDHRA for all three RAR isotypes. ESI-MS experiments performed with hRARα, β and γ LBDs (Supporting Fig. S3 a and b) revealed that 9CDHRA (R and S) bind to all RAR LBD isotypes.

To provide structural evidence of the binding of (*R*)-9CDHRA to RXR, the hRXRα LBD was crystallized in complex with (*R*)-9CDHRA and a 13-residue peptide comprising the nuclear receptor-binding surface NR2 of NCoA2. Note that the residues lining the ligand-binding pocket are strictly conserved between the RXRα and RXRγ and that the conclusions drawn for RXRα will also be valid for RXRγ. The structure refined at 1.8 Å resolution (Supporting Table S1) revealed the canonical active agonist conformation common to all previously reported agonist-bound nuclear receptor LBDs with 12 or 13 α-helices organized in a three-layered sandwich (Supporting Figs. S3c and d). (*R*)-9CDHRA adopts a similar binding mode as 9CRA [29,30] including interactions of carboxyl groups of the ligand with Arg316 (H5), and hydrogen bonds with the amide group of Ala327 in the beta turn (Figs. 3c and d). The number of contacts is similar between the two ligands although some interactions are weaker in the case of (*R*)-9CDHRA compared to 9CRA as for example the interactions with Leu436 (4.0 Å instead of 3.6 Å for 9CRA), Arg316 (2.7 Å instead of 2.3 Å) or Trp305 (4.3 Å instead of 3.5 Å) that account for the weaker binding of the (*R*)-9CDHRA compound. In silico comparison of (S)- and (*R*)-9CDHRA binding mode in RXRα LBP revealed that the opposite configuration at C13 leading to slightly different side chain conformation of (*S*)-9CDHRA may underlay its lower affinity to RXR (Supporting Fig. S3e), further supported by the lower relative binding affinity measured by ESI-MS.

The relevance of (*R*)-9CDHRA and (*S*)-9CDHRA receptor binding for transcriptional activities of RXRs was tested in COS1 reporter cell lines transfected with a RXRα expression vector (Figs. 3e-g). In agreement with previous reports, 9CRA induced transcription of reporter gene at concentrations starting from 10⁻⁹M, whereas (*R*)-9CDHRA or (*S*)-9CDHRA displayed similar activity to 9CRA, although at concentrations higher than 10⁻⁷M. Importantly, the activities of (R)- and (*S*)-9CDHRA at 10⁻⁵M were prevented by co-treatment with an RXR-antagonist LG101208 at 10⁻⁶M (Fig. 3f). 9CDHRAs also activated RAR-RXR signaling in COS1 model reporter cells (transfected with RARα and RXRα expression vectors) starting at 10⁻⁶M (Fig. 3g). Considering that all RXR isotypes share the same structure of ligand binding pocket, present data obtained with RXRα isotype indicate that 9CDHRA may efficiently bind to all RXRs and induce their transcriptional activities at concentrations found in physiological conditions.

Behavioral analyses revealed that 9CDHRA modulation of RXR functions is also relevant *in vivo.* Accordingly, acute treatment with (*R*)-9CDHRA improved memory performance of *Rbp1*^{*-*/*-*} mice as compared to vehicle treatment or chance level of 50% when tested in DNMTP task at ITI of 6 min (Fig. 3h). (*R*)-9CDHRA treatments also raised performance of WT mice when tested at long ITIs of 12 or 18 min, at which the corresponding WT mice treated with vehicle performed at chance level. Such treatment did not improve performance of *Rxrγ*^{*-*/*-*} mice (57 ± 7% of correct choices) indicating RXR specificity of 9CDHRA effects, which is further supported by similar effects of 9CDHRA and UVI2108 treatments (compare Figs. 3h and 1).

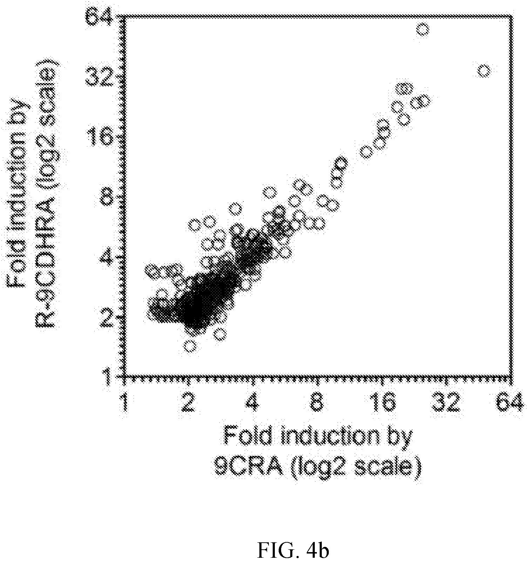

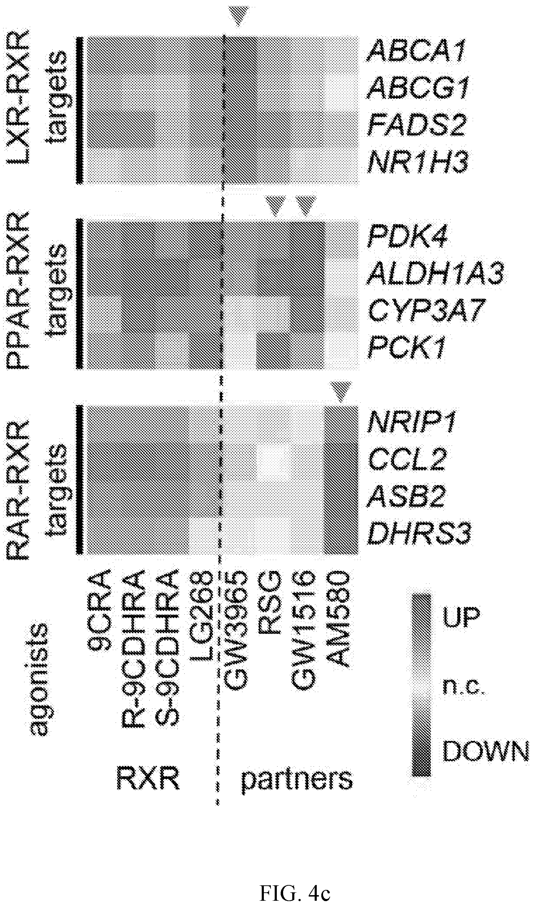

In order to identify 9CDHRA specificity for induction of RXR-dependent transcriptional activity at the transcriptomic level and its capacity to activate permissive heterodimers inventors took advantage of human differentiating monocyte-derived dendritic cell cultures, a well characterized *in vitro* model for studies of signaling through RXR and its heterodimers [31,32]. The gene expression changes induced by (*R*)-9CDHRA, (*S*)-9CDHRA, other RXR ligands or ligands for RXR partners, revealed that (*R*)-9CDHRA and 9CRA regulate approximately the same number of transcripts (518 and 450, respectively; Fig. 4). Importantly, 384 transcripts were similarly regulated by both agonists (Figs. 4a, b), which corresponded to 85% of all transcripts regulated by 9CRA. Within this set, a group of 61 transcripts was also regulated by LG268, a synthetic RXR specific ligand used in our analysis as a reference to previous studies of this model [31,32]. Remarkably, none of the transcripts were regulated solely by 9CRA and LG268, and not by 9CDHRA, indicating that 9CDHRA induces similar gene expression changes as 9CRA.

Inventors also investigated the capacity of 9CDHRA for activating permissive heterodimers, e.g. LXRα/β-RXR, PPARγ-RXR, and PPARδ-RXR. As expected, inventors found that (*R*)-9CDHRA similarly to 9CRA and LG268 could induce the expression of many genes, which are known as direct targets of RXR permissive heterodimers. Accordingly these genes were also regulated by LXR or PPAR specific ligands (Fig. 4c). To address whether 9CDHRA also activates RAR-RXR target genes inventors compared the effect of RXR ligands and AM580, a synthetic RARα selective ligand. Typically genes induced by AM580 were not induced by any other agonist for permissive partners (Fig. 4c), but were also induced by 9CDHRA or 9CRA. Collectively, gene expression profiling indicated that (R)- and (*S*)-9CDHRAs display RXR agonist activity, but can also activate RARs, acting thus with similar selectivity to 9CRA.

Although RXRs occupy central position in signaling of several nuclear hormone receptors acting as their heterodimerisation partner, endogenous ligand(s) of RXRs, its metabolic pathway and physiological functions were not conclusively determined. We found that *Rbp1*^{*-*/*-*} displayed a phenotype suggestive of reduced RXR signaling, which could not be attributed to the reduced levels of RXR expression or of 9CRA, the potential endogenous RXR ligand which we and others failed to detect [11, 12, 13, 14, 15] in wild type animals. Using chemical approaches of HPLC-MS and organic synthesis we identified 9CDHRA, a novel endogenous retinoid, the concentrations of which were significantly reduced in serum, liver and brain of *Rbp1*^{*-*/*-*} mice. Several lines of evidence indicate that 9CDHRA treatment activates RXRs *in vitro* and *in vivo* at physiologically relevant concentrations, suggesting that it acts as an endogenous RXR agonist.

### Conclusion:

The inventors report herein that RBP1 modulates animal behavior by control of the availability of an RXR ligand. Accordingly, mice carrying null mutation of RBP1 display working memory deficits, the hallmark of deficient signalling through RXRγ, a functionally predominant RXR in the control of working memory in adult mice [4, 59]. Reduced expression of RXRγ or other RXRs (data not shown) do not account for these changes, suggesting compromised availability of RXR ligand. This unique model enabled to search for the endogenous RXR ligand(s). As the initial analyses failed to detect 9CRA in wild type and *Rbp1^{-l-}* mice, inventors turned their attention to dihydroretinoids proposed recently as a novel group of bioactive, endogenous retinoids [26]. Using HPLC-MS-MS conditions specific for detection of dihydroretinoic acids, including 13,14-dihydroretinoic acids, and aided by organic synthesis inventors detected the presence of ATDHRA and 9CDHRA in mouse serum, liver and brain in WT mice and *Rbp1*^{*-*/*-*} mice. Serum and liver concentration of 9CDHRA were particularly high, ranging from 4 to 7x10⁻⁷M, and much lower in whole-brain extracts in WT animals. Nevertheless they were significantly reduced in all corresponding samples of *Rbp1*^{*-*/*-*} mice.

Reduced serum availability of 9CDHRA in *Rbp1*^{*-*/*-*} mice may result from reduced synthesis of 9CDHRA in the liver, the main site of RBP1 expression [24]. Because levels of ATDHRA were comparable in the serum, liver and brain of WT and *Rbp1^{-l-}* mice, reduced levels of 9CDHRA in *Rbp1^{-l-}* mice indicate that RBP1 plays an important role specifically in generation of different forms of 9-cis-retinoids as previously suggested [60]. Thus, 9CDHRA, similarly to ATRA and 1,25-dihydroxy-vitamin D3 could act in endocrine and paracrine manner as a lipid hormone of nutritional origin being distributed in the serum but also synthetized locally in specific organs [61; 62]. In consequence, reduced systemic levels of 9CDHRA may synergize with local reduction of its synthesis in specific brain areas of *Rbp1*^{*-*/*-*} mice leading to compromised RXR activities and mnemonic deficits. In favor of this hypothesis, systemic administration of R-9CDHRA, a 9CDHRA enantiomer obtained by stereoselective chemical synthesis, normalized working memory deficits in *Rbp1*^{*-*/*-*} mice. That such effects are mediated by RXRs may be suggested by absence of promnemonic effects of 9CDHRA and other RXR ligands in mice carrying null mutation of RXRγ, a functionally predominant RXR in the control of these brain functions [73].

Direct evidence of 9CDHRA binding to RXRs is provided by electrospray ionisation mass spectrometry (ESI-MS) performed in non-denaturing conditions using purified RXR LBD and fluorescence quenching assay. In particular, R-9CDHRA binds RXR LBD with affinity close to that of 9CRA as indicated by respective Kd values of 90 ± 20 nM for 9CDHRA and 20 ± 10 nM for 9CRA. Such data are supported by the crystal structure of the complex of R-9CDHRA with RXR LBD, in which R-9CDHRA adopts the canonical active agonist conformation and the carboxylate interacts with Arg316. Importantly, the R-9CDHRA enantiomer efficiently induces RXR transcriptional activity in reporter cell assays at physiologically relevant concentrations below 10⁻⁶M, which can be prevented by co-administration of RXR pan-antagonist LG101208. Although S-9CDHRA displays lower affinity to bind RXR LBD in ESI-MS, most probably due to the inverse positioning of the C20-carbon atom, it is also active in the transactivation of RXR in vitro with threshold concentration between 10⁻⁷ and 10⁻⁶M.

Further relevance of 9CDHRA for the activation of RXRs *in vitro* was indicated by the regulation of transcriptional targets of LXR-RXR or PPAR-RXR permissive heterodimers, which are known to be sensitive to pharmacological activation of RXR as well as its nuclear receptor partners. Such activation was demonstrated in human dendritic cells cultured *in vitro,* a well-established model for analyses of RXR signalling [3, 48]. Importantly, almost all (68 out of 72) transcripts regulated by LG268 (RXR-specific agonist) were also regulated by 9CDHRA. Such a result obtained in a course of transcriptomics study was very similar to the data obtained for 9CRA, which controlled 61 out 72 LG268 transcriptional targets, indicating the extremely high capacity of 9CDHRA and 9CRA to control RXR transcriptional targets. Such genes correspond most probably to permissive heterodimers, and indirect targets of liganded RXR and their regulation provide evidence that 9CDHRA can control RXR signalling also in human cells. High degree of overlap between transcriptional activities of 9CRA and 9CDHRA, which goes beyond the activation of RXR-specific transcripts, reflects their capacity to bind and transactivate also RARs. That 9CRA and 9CDHRA act as a mixed RXR and RAR agonists is supported by about 80% overlap in transcriptional changes induced by R-9CDHRA (or S-9CDHRA) and 9CRA.

Whereas 13,14-dihydroretinol was detected by Moise and colleagues [26] as a hepatic retinol saturase (RETSAT) metabolite in the mammalian organism, other dihydroretinoids or their precursors were also identified in other vertebrates [63, 64] and also non-vertebrates [65]. All-trans-13,14-dihydroretinol saturase has also been described by Moise and colleagues (WO2006/029398). Besides RETSAT-mediated retinol metabolism as the major potential pathway for endogenous 9CDHRA synthesis, also apo-carotenoids and carotenoids may serve as substrates for hydrogenation via RETSAT or other saturases, followed by the synthesis of dihydroretinoic acids [66].

This metabolic pathway may be phylogenetically ancient, as RXR orthologs from several non-vertebrate species including mollusk [65], primitive chordate like amphioxus [69] and some primitive insects like *Tribolium* [70] or *Locusta migratoria* [71; 72] have also a potential to bind RXR ligands. In addition, *Locusta migratora* ultraspiracle (a fly RXR ortholog), displayed higher affinity to bind 9CRA than human RXR [72], raising the possibility that 9CDHRA could also activate the USP pathway and be an ancestral RXR ligand. Based on the data, it is tempting to suggest that in addition to the active ligands originating from vitamin A1 and vitamin A2, 9CDHRA and its nutritional precursors may represent a novel distinct pathway of vitamin A metabolism and signaling, which evolved specifically to control RXR activity.

In summary inventors characterized 9CDHRA as the first endogenous and physiologically relevant retinoid that acts as RXR ligand in mammals. Reduced memory functions due to compromised RXR-mediated signaling in *Rbp1*^{*-*/*-*} mice result from lower brain levels of 9CDHRA reflecting reduced serum transport and local brain synthesis of 9CDHRA in *Rbp1*^{*-*/*-*} mice. Future determination of the metabolic pathways involved in 9CDHRA synthesis and signalling and its tissue specificity will be important for further understanding of functional relevance of 9CDHRA for animal physiology and pathology.

### REFERENCES

1. Perez E, Bourguet W, Gronemeyer H, de Lera AR (2012) Modulation of RXR function through ligand design. Biochim Biophys Acta 1821: 57-69.
2. Shulman AI, Larson C, Mangelsdorf DJ, Ranganathan R (2004) Structural determinants of allosteric ligand activation in RXR heterodimers. Cell 116: 417-429.
3. Altucci L, Leibowitz MD, Ogilvie KM, de Lera AR, Gronemeyer H (2007) RAR and RXR modulation in cancer and metabolic disease. Nat Rev Drug Discov 6: 793-810.
4. Wietrzych-Schindler M, Szyszka-Niagolov M, Ohta K, Endo Y, Perez E, et al. (2011) Retinoid x receptor gamma is implicated in docosahexaenoic acid modulation of despair behaviors and working memory in mice. Biol Psychiatry 69: 788-794.
5. Huang JK, Jarjour AA, Nait Oumesmar B, Kerninon C, Williams A, et al. (2011) Retinoid X receptor gamma signaling accelerates CNS remyelination. Nat Neurosci 14: 45-53.
6. Lerner V, Miodownik C, Gibel A, Kovalyonok E, Shleifer T, et al. (2008) Bexarotene as add-on to antipsychotic treatment in schizophrenia patients: a pilot open-label trial. Clin Neuropharmacol 31: 25-33.
7. de Urquiza AM, Liu S, Sjoberg M, Zetterstrom RH, Griffiths W, et al. (2000) Docosahexaenoic acid, a ligand for the retinoid X receptor in mouse brain. Science 290: 2140-2144.
8. Heyman RA, Mangelsdorf DJ, Dyck JA, Stein RB, Eichele G, et al. (1992) 9-cis retinoic acid is a high affinity ligand for the retinoid X receptor. Cell 68: 397-406.
9. Levin AA, Sturzenbecker LJ, Kazmer S, Bosakowski T, Huselton C, et al. (1992) 9-cis retinoic acid stereoisomer binds and activates the nuclear receptor RXR alpha. Nature 355: 359-361.
10. Allenby G, Bocquel MT, Saunders M, Kazmer S, Speck J, et al. (1993) Retinoic acid receptors and retinoid X receptors: interactions with endogenous retinoic acids. Proc Natl Acad Sci U S A 90: 30-34.
11. Sakhi AK, Gundersen TE, Ulven SM, Blomhoff R, Lundanes E (1998) Quantitative determination of endogenous retinoids in mouse embryos by high-performance liquid chromatography with on-line solid-phase extraction, column switching and electrochemical detection. J Chromatogr A 828: 451-460.
12. Ulven SM, Gundersen TE, Sakhi AK, Glover JC, Blomhoff R (2001) Quantitative axial profiles of retinoic acid in the embryonic mouse spinal cord: 9-cis retinoic acid only detected after all-trans-retinoic acid levels are super-elevated experimentally. Dev Dyn 222: 341-353.
13. Kane MA, Chen N, Sparks S, Napoli JL (2005) Quantification of endogenous retinoic acid in limited biological samples by LC/MS/MS. Biochem J 388: 363-369.
14. Gundersen TE, Bastani NE, Blomhoff R (2007) Quantitative high-throughput determination of endogenous retinoids in human plasma using triple-stage liquid chromatography/tandem mass spectrometry. Rapid Commun Mass Spectrom 21: 1176-1186.
15. Rühl R (2006) Method to determine 4-oxo-retinoic acids, retinoic acids and retinol in serum and cell extracts by liquid chromatography/diode-array detection atmospheric pressure chemical ionisation tandem mass spectrometry. Rapid Commun Mass Spectrom 20: 2497-2504.
16. Wolf G (2006) Is 9-cis-retinoic acid the endogenous ligand for the retinoic acid-X receptor? Nutr Rev 64: 532-538.
17. Arnold SL, Amory JK, Walsh TJ, Isoherranen N (2012) A sensitive and specific method for measurement of multiple retinoids in human serum with UHPLC-MS/MS. J Lipid Res 53: 587-598.
18. Fan YY, Spencer TE, Wang N, Moyer MP, Chapkin RS (2003) Chemopreventive n-3 fatty acids activate RXRalpha in colonocytes. Carcinogenesis 24: 1541-1548.
19. Egea PF, Mitschler A, Moras D (2002) Molecular recognition of agonist ligands by RXRs. Mol Endocrinol 16: 987-997.
20. Elabdeen HR, Mustafa M, Szklenar M, Ruhl R, Ali R, et al. (2013) Ratio of proresolving and pro-inflammatory lipid mediator precursors as potential markers for aggressive periodontitis. PLoS One 8: e70838.
21. Szklenar M, Kalkowski J, Stangl V, Lorenz M, Ruhl R (2013) Eicosanoids an docosanoids in plasma and aorta of healthy and atherosclerotic rabbits. J Vasc Res 50: 372-382.
22. Kitareewan S, Burka LT, Tomer KB, Parker CE, Deterding LJ, et al. (1996) Phytol metabolites are circulating dietary factors that activate the nuclear receptor RXR. Mol Biol Cell 7: 1153-1166.
23. Lemotte PK, Keidel S, Apfel CM (1996) Phytanic acid is a retinoid X receptor ligand. Eur J Biochem 236: 328-333.
24. Ghyselinck NB, Bavik C, Sapin V, Mark M, Bonnier D, et al. (1999) Cellular retinolbinding protein I is essential for vitamin A homeostasis. Embo J 18: 4903-4914.
25. Duell EA, Kang S, Voorhees JJ (1996) Retinoic acid isomers applied to human skin in vivo each induce a 4-hydroxylase that inactivates only trans retinoic acid. J Invest Dermatol 106: 316-320.
26. Moise AR, Kuksa V, Imanishi Y, Palczewski K (2004) Identification of all-transretinol: all-trans-13,14-dihydroretinol saturase. J Biol Chem 279: 50230-50242.
27. Shirley MA, Bennani YL, Boehm MF, Breau AP, Pathirana C, et al. (1996) Oxidative and reductive metabolism of 9-cis-retinoic acid in the rat. Identification of 13,14-dihydro-9-cis-retinoic acid and its taurine conjugate. Drug Metab Dispos 24: 293-302.
28. Lein ES, Hawrylycz MJ, Ao N, Ayres M, Bensinger A, et al. (2007) Genome-wide atlas of gene expression in the adult mouse brain. Nature 445: 168-176.
29. Egea PF, Mitschler A, Rochel N, Ruff M, Chambon P, et al. (2000) Crystal structure of the human RXRalpha ligand-binding domain bound to its natural ligand: 9-cis-retinoic acid. Embo J 19: 2592 2601.
30. Pogenberg V, Guichou JF, Vivat-Hannah V, Kammerer S, Perez E, et al. (2005) Characterization of the interaction between retinoic acid receptor/retinoid X receptor (RAR/RXR) heterodimers and transcriptional coactivators through structural and fluorescence anisotropy studies. J Biol Chem 280: 1625-1633.
31. Szatmari I, Torocsik D, Agostini M, Nagy T, Gurnell M, et al. (2007) PPARgamma regulates the function of human dendritic cells primarily by altering lipid metabolism. Blood 110: 3271-3280.
32. Szeles L, Poliska S, Nagy G, Szatmari I, Szanto A, et al. (2010) Research resource: transcriptome profiling of genes regulated by RXR and its permissive and nonpermissive partners in differentiating monocyte-derived dendritic cells. Mol Endocrinol 24: 2218-2231.
33. Krezel W, Dupe V, Mark M, Dierich A, Kastner P, et al. (1996) RXR gamma null mice are apparently normal and compound RXR alpha +/-/RXR beta -/-/RXR gamma -/- mutant mice are viable. Proc Natl Acad Sci U S A 93: 9010-9014.
34. Wietrzych M, Meziane H, Sutter A, Ghyselinck N, Chapman PF, et al. (2005) Working memory deficits in retinoid X receptor gamma-deficient mice. Learn Mem 12: 318-326.
35. Nagy L, Kao HY, Love JD, Li C, Banayo E, et al. (1999) Mechanism of corepressor binding and release from nuclear hormone receptors. Genes Dev 13: 3209-3216.
36. Lippert WP, Burschka C, Gotz K, Kaupp M, Ivanova D, et al. (2009) Silicon analogues of the RXR-selective retinoid agonist SR11237 (BMS649): chemistry and biology. ChemMedChem 4: 1143-1152.
37. Osz J, Brelivet Y, Peluso-Iltis C, Cura V, Eiler S, et al. (2012) Structural basis for a molecular allosteric control mechanism of cofactor binding to nuclear receptors. Proc Natl Acad Sci U S A 109: E588-594.
38. Pazos Y, Iglesias B, de Lera AR (2001) The Suzuki coupling reaction in the stereocontrolled synthesis of 9-cis-retinoic acid and its ring-demethylated analogues. J Org Chem 66: 8483-8489.
39. Brunger AT (1992) Free R value: a novel statistical quantity for assessing the accuracy of crystal structures. Nature 355: 472-475.
41. Wietrzych M, Meziane H, Sutter A, Ghyselinck N, Chapman PF, et al. (2005) Working memory deficits in retinoid X receptor gamma-deficient mice. Learn Mem 12: 318-326.
42. Rühl R (2006) Method to determine 4-oxo-retinoic acids, retinoic acids and retinol in serum and cell extracts by liquid chromatography/diode-array detection atmospheric pressure chemical ionisation tandem mass spectrometry. Rapid Commun Mass Spectrom 20: 2497-2504.
43. Nagy L, Kao HY, Love JD, Li C, Banayo E, et al. (1999) Mechanism of corepressor binding and release from nuclear hormone receptors. Genes Dev 13: 3209-3216.
44. Lippert WP, Burschka C, Gotz K, Kaupp M, Ivanova D, et al. (2009) Silicon analogues of the RXR-selective retinoid agonist SR11237 (BMS649): chemistry and biology. ChemMedChem 4: 1143-1152.
45. Osz J, Brelivet Y, Peluso-Iltis C, Cura V, Eiler S, et al. (2012) Structural basis for a molecular allosteric control mechanism of cofactor binding to nuclear receptors. Proc Natl Acad Sci U S A 109: E588-594.
46. Otwinowski Z, Minor W, editors (1997) Processing of X-ray Diffraction Data Collected in Oscillation Mode. Macromolecular Crystallography, part A ed. New York: Academic Press. 307-326 p.
47. Wietrzych-Schindler M, Szyszka-Niagolov M, Ohta K, Endo Y, Perez E, et al. (2011) Retinoid x receptor gamma is implicated in docosahexaenoic acid modulation of despair behaviors and working memory in mice. Biol Psychiatry 69: 788-794.
48. Szeles L, Poliska S, Nagy G, Szatmari I, Szanto A, et al. (2010) Research resource: transcriptome profiling of genes regulated by RXR and its permissive and nonpermissive partners in differentiating monocyte-derived dendritic cells. Mol Endocrinol 24: 2218-2231.
49. Dominguez B, Pazos Y, de Lera AR (2000) Stereocontrolled Synthesis of 6-s-cis and 6-s-trans-Locked 9Z-Retinoids by Hydroxyl-Accelerated Stille Coupling of (Z)-Tri-n-Butylstannylbut-2-en-1-ol and Bicyclic Dienyl Triflates. J Org Chem 65: 5917-5925.
50. Pazos Y, de Lera AR (1999) Stereoselective Synthesis of 9-cis-Retinoic Acid Based on Stepwise or Convergent Suzuki Coupling Reactions. Tetrahedron Lett 40: 8287-8290.
51. Schultz HS, Freyermuth HB, Buc SR (1963) New Catalysts for the Oxidation of Sulfides to Sulfones with Hydrogen Peroxide. J Org Chem 28: 1140-1142.
52. Blakemore PR (2002) The modified Julia olefination: alkene synthesis via the condensation of metallated heteroarylalkylsulfones with carbonyl compounds. J Chem Soc Perkin Trans 1: 2563-2585.
53. Aïssa C (2009) Mechanistic Manifold and New Development of the Julia-Kocienski Reaction. Eur J Org Chem: 1831-1844.
54. Moise AR, Dominguez M, Alvarez S, Alvarez R, Schupp M, et al. (2008) Stereospecificity of Retinol Saturase: Absolute Configuration, Synthesis, and Biological Evaluation of Dihydroretinoids. J Am Chem Soc 130: 1154-1155.
55. Leonard J, Mohialdin S, Reed D, Ryan G, Swain PA (1995) Stereoselective conjugate addition of organolithium and organocopper reagents to [delta]-oxygenated [alpha],[beta]-unsaturated carbonyl systems derived from glyceraldehyde acetonide. Tetrahedron 51: 12843-12858.
56. Sorg A, Brückner R (2005) Unexpected cis-Selectivity in (Sylvestre) Julia Olefinations with Bu3Sn-Containing Allyl Benzothiazolyl Sulfones: Stereoselective Synthesis of 1,3-Butadienyl- and 1,3,5-Hexatrienylstannanes. Synlett 2005: 289,293.
57. Vaz B, Alvarez R, Souto JA, de Lera AR (2005) γ-Allenyl Allyl Benzothiazole Sulfonyl Anions Undergo cis-Selective (Sylvestre) Julia Olefinations. Synlett 2005: 294,298.
58. Ghyselinck NB, Bavik C, Sapin V, Mark M, Bonnier D, et al. (1999) Cellular retinol-binding protein I is essential for vitamin A homeostasis. Embo J 18: 4903-4914.
59. Krzyzosiak A, Szyszka-Niagolov M, Wietrzych M, Gobaille S, Muramatsu S, et al. (2010) Retinoid x receptor gamma control of affective behaviors involves dopaminergic signaling in mice. Neuron 66: 908-920.
60. Kane MA, Bright FV, Napoli JL (2011) Binding affinities of CRBPI and CRBPII for 9-cis-retinoids. Biochim Biophys Acta 1810: 514-518.
61. Prosser DE, Jones G (2004) Enzymes involved in the activation and inactivation of vitamin D. Trends Biochem Sci 29: 664-673.
62. Rühl R, Bub A, Watzl B (2008) Modulation of plasma all-trans retinoic acid concentrations by the consumption of carotenoid-rich vegetables. Nutrition 24: 1224-1226.
63. Aydemir G, Kasiri Y, Birta E, Beke G, Garcia AL, et al. (2013) Lycopene-derived bioactive retinoic acid receptors/retinoid-X receptors-activating metabolites may be relevant for lycopene's anti-cancer potential. Mol Nutr Food Res 57: 739-747.
64. Moise AR, Isken A, Dominguez M, de Lera AR, von Lintig J, et al. (2007) Specificity of zebrafish retinol saturase: formation of all-trans-13,14-dihydroretinol and all-trans-7,8- dihydroretinol. Biochemistry 46: 1811-1820.
65. Foster JM, Pennock JF, Marshall I, Rees HH (1993) Biosynthesis of isoprenoid compounds in Schistosoma mansoni. Mol Biochem Parasitol 61: 275-284.
66. Gouranton E, Aydemir G, Reynaud E, Marcotorchino J, Malezet C, et al. (2011) Apo-10'-lycopenoic acid impacts adipose tissue biology via the retinoic acid receptors. Biochim Biophys Acta 1811: 1105-1114.
67. Sicilia T, Bub A, Rechkemmer G, Kraemer K, Hoppe PP, et al. (2005) Novel lycopene metabolites are detectable in plasma of preruminant calves after lycopene supplementation. J Nutr 135: 2616-2621.
68. De Groot A, de Rosny E, Juillan-Binard C, Ferrer JL, Laudet V, et al. (2005) Crystal structure of a novel tetrameric complex of agonist-bound ligand-binding domain of Biomphalaria glabrata retinoid X receptor. J Mol Biol 354: 841-853.
69. Tocchini-Valentini GD, Rochel N, Escriva H, Germain P, Peluso-Iltis C, et al. (2009) Structural and functional insights into the ligand-binding domain of a nonduplicated retinoid X nuclear receptor from the invertebrate chordate amphioxus. J Biol Chem 284: 1938-1948.
70. Iwema T, Billas IM, Beck Y, Bonneton F, Nierengarten H, et al. (2007) Structural and functional characterization of a novel type of ligand-independent RXR-USP receptor. Embo J 26: 3770-3782.
71. Nowickyj SM, Chithalen JV, Cameron D, Tyshenko MG, Petkovich M, et al. (2008) Locust retinoid X receptors: 9-Cis-retinoic acid in embryos from a primitive insect. Proc Natl Acad Sci U S A 105: 9540-9545.
72. Palli SR, Kapitskaya MZ, Potter DW (2005) The influence of heterodimer partner ultraspiracle/retinoid X receptor on the function of ecdysone receptor. FEBS J 272: 5979-5990.
73. Wietrzych-Schindler M, Szyszka-Niagolov M, Ohta K, Endo Y, Perez E, de Lera AR, Chambon P, Krezel W (2011) Retinoid x receptor gamma is implicated in docosahexaenoic acid modulation of despair behaviors and working memory in mice. Biol Psychiatry 69:788-794.
74. Chen ZP, Shemshedini L, Durand B, Noy N, Chambon P, et al. (1994) Pure and functionally homogeneous recombinant retinoid X receptor. J Biol Chem 269: 25770-25776.
75. Berton Ol, McClung CA, Dileone RJ, Krishnan V, Renthal W, Russo SJ, Graham D, Tsankova NM, Bolanos CA, Rios M, Monteggia LM, Self DW, Nestler EJ. (2006) Essential role of BDNF in the mesolimbic dopamine pathway in social defeat stress. Science. 311(5762):864-8.
76. Moreau.J.L. (1997) Reliable monitoring of hedonic deficits in the chronic mild stress model of depression. Psychopharmacology 134:357-358.
77. Dalvi A, Lucki I. (1999) Murine models of depression. Psychopharmacology 147:14-16
78. Hollis F, Kabbaj M. (2014) Social defeat as an animal model for depression. ILAR J.55(2):221-32.

## Claims

1. An enantioselective method for the preparation of an R or S enantiomer compound selected from the group consisting of R or S 9-cis-13,14-dihydroretinoic acid or esters thereof according to general formula I, a solvate and, if appropriate, a salt thereof, said method comprising 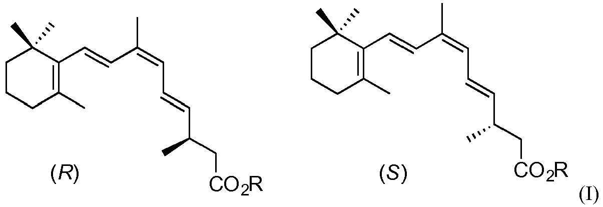 wherein in said formula R is selected from H or Ethyl;
- providing the respective 9Z,11E geometric isomer of R or S enantiomer of trienyliodide of Formula 3 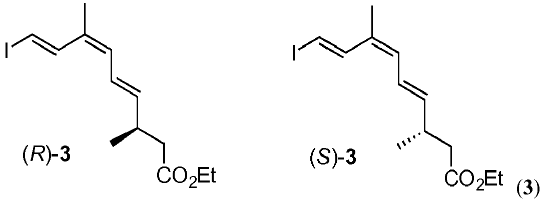
- reacting, respectively, said R or S enantiomer of trienyliodide of Formula 3 with boronic acid of Formula 2 by Suzuki coupling to obtain said compound as R or S ethyl-9-cis-13,14-dihydroretinoate of Formula (I); 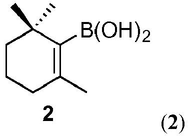
- optionally saponifying said R or S ethyl-9-cis-13,14-dihydroretinoate to obtain said compound as R or S 9-cis-13,14-dihydroretinoic acid, respectively;
- optionally forming said compound into a solvate or, if appropriate, salt thereof.

2. The method according to claim 1 wherein
the R or S enantiomer of trienyliodide of Formula 3 is prepared from the stannane of Formula 9 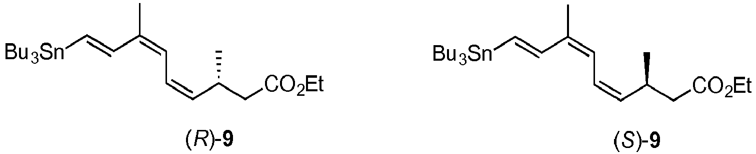 with a solution of iodine in solvent, preferably CH₂Cl₂, via Sn-I exchange and iodine-promoted isomerization of the 9Z,11Z diene to the desired 9Z,11E geometric isomer.

3. The method according to claim 2 further comprising the steps of
(a) transforming the stannyldienol of Formula 5 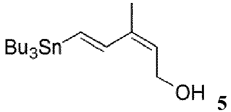 by Mitsunobu reaction with the corresponding thiol into benzothiazolyl allyl sulfide having Formula 6 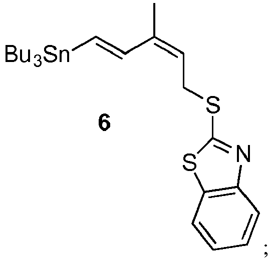
(b) oxidizing the benzothiazolyl allyl sulfide having Formula 6 to the corresponding sulfone having Formula 7 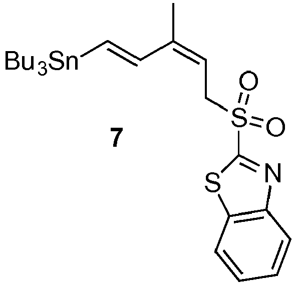 with hydrogen and a peroxymolybdate (VI) reagent;
- (c) reacting the sulfone having formula 7 with R or S enantiomer of aldehyde 8, respectively, 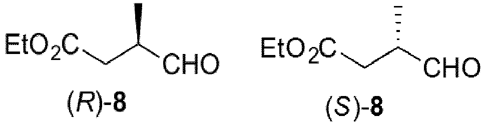
in the presence of base by Julia-Kocienski olefination;
to obtain the stannane of Formula 9 as defined in claim 2.

4. The method of any of claims 1 to 3 wherein the enatiomers are enantiomerically pure compounds.

5. An enantiomer compound of 9-*cis*-13,14-dihydroretinoic acid or an ester precursor thereof wherein said compound is selected from the group consisting of
(*R*)-9-*cis*-13,14-dihydroretinoic acid,
(*S*)- *9-cis-13,14-dihydroretinoic* acid
(9Z,13R)-ethyl-13,14-dihydroretinoate((R)-4) and
(9Z,13R)-ethyl 13,14-dihydroretinoate ((S)-4), and
solvates, solid forms and, if appropriate, salts thereof.

6. An enantiomer compound of 9-*cis*-13,14-dihydroretinoic acid
wherein said compound is selected from the group consisting of
(*R*)-9-*cis*-13,14-dihydroretinoic acid,
(*S*)- 9-*cis*-13,14-dihydroretinoic acid, and
solvates, solid forms and salts thereof.

7. An enantiomer compound (*R*)-9-*cis*-13,14-dihydroretinoic acid or a solvate, solid form or salt thereof.

8. The enantiomer compound according to any of claims 4 to 6 wherein the compound is in an enantiomerically pure form.

9. A pharmaceutical composition comprising an enantiomerically pure compound selected from the enantiomer compounds according to claim 5, solvates, solid forms and, if appropriate, salts thereof,
in a pharmaceutically acceptable carrier.

10. A pharmaceutical composition comprising an enantiomerically pure compound of 9-*cis*-13,14-dihydroretinoic acid,
wherein said compound is selected from the group consisting of
*(R)-9-cis-13,14-dihydroretinoic* acid,
(*S*)-9-*cis*-13,14-dihydroretinoic acid,
solvates, salts and solid forms thereof,
in a pharmaceutically acceptable carrier.

11. An enantiomer compound of 9-*cis*-13,14-dihydroretinoic acid,
wherein said compound is selected from the group consisting of
*(R)-9-cis-13,14-dihydroretinoic* acid,
(*S*)- *9-cis-13,14-dihydroretinoic* acid
or a pharmaceutical composition according to claim 8,
for use in a therapeutic method.

12. The enantiomer compound or the pharmaceutical composition for use according to claim 11 for use in the treatment of a psychiatric disorder.

13. The enantiomer compound or the pharmaceutical composition for use according to claim 11 for use in the prevention and/or treatment of memory impairment or for use in enhancing memory performance, wherein preferably said memory is working memory, or for use in the prevention and/or treatment of impaired cognitive functions or impaired learning, or for use in the treatment of depression.

14. (*R*)-9-*cis*-13,14-dihydroretinoic acid for use in the prevention and/or treatment of memory impairment or for use in enhancing memory performance, wherein preferably said memory is working memory, or for use in the prevention and/or treatment of impaired cognitive functions or impaired learning, or for use in the treatment of depression.

15. A functional food or a dietary supplement comprising one or more enantiomerically pure compound(s) according to any of claims 4-6.

## Patentansprüche

1. Enantioselektives Verfahren zur Herstellung einer R- oder S-Enantiomerenverbindung ausgewählt aus der Gruppe bestehend aus R- oder S-9-cis-13,14-Dihydro-Retinsäure oder deren Estern gemäß der allgemeinen Formel I, einem Solvat und gegebenenfalls einem Salz davon, wobei das Verfahren 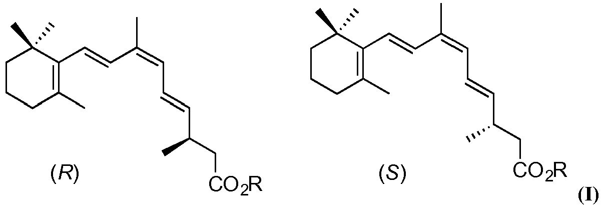 umfasst,
wobei in der Formel R aus H oder Ethyl ausgewählt ist;
- Bereitstellen des jeweiligen geometrischen 9Z,11E-Isomers von R- oder S-Enantiomer von Trienyliodid der Formel 3 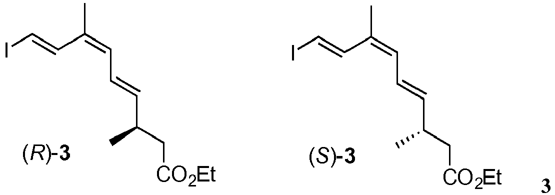
- Umsetzen jeweils des R- oder S-Enantiomers von Trienyliodid der Formel 3 mit Boronsäure der Formel 2 durch Suzuki-Kupplung, um die Verbindung als R- oder S-Ethyl-9-cis-13,14-Dihydro-Retinoat der Formel (I) zu erhalten; 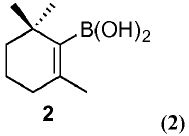
- gegebenenfalls Verseifen des R- oder S-Ethyl-9-cis-13,14-Dihydro-Retinoats, um die Verbindung jeweils als R- oder S-9-cis-13,14-Dihydro-Retinsäure zu erhalten;
- gegebenenfalls Formen der Verbindung zu einem Solvat oder gegebenenfalls Salz davon.

2. Verfahren nach Anspruch 1, wobei
das R- oder S-Enantiomer von Trienyliodid der Formel 3 aus dem Stannan der Formel 9 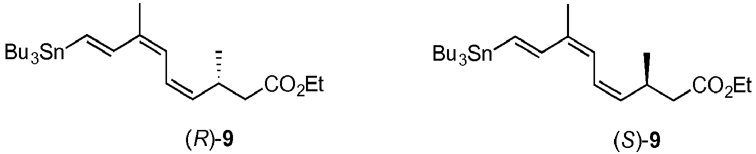 mit einer Lösung von Iod in Lösungsmittel, vorzugsweise CH₂Cl₂, über Sn-I-Austausch und Iod-beförderte Isomerisierung des 9Z,11Z-Diens zum gewünschten geometrischen 9Z,11E-Isomer hergestellt wird.

3. Verfahren nach Anspruch 2, ferner umfassend die Schritte von
(a) Transformieren des Stannyldienols der Formel 5 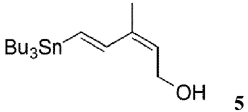 durch Mitsunobu-Reaktion mit dem entsprechenden Thiol zu Benzothiazolyl-Allylsulfid mit der Formel 6 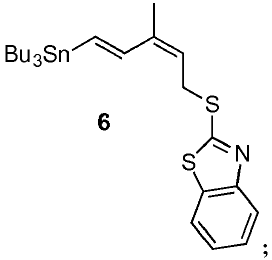
(b) Oxidieren des Benzothiazolyl-Allylsulfids mit der Formel 6 zu dem entsprechenden Sulfon mit der Formel 7 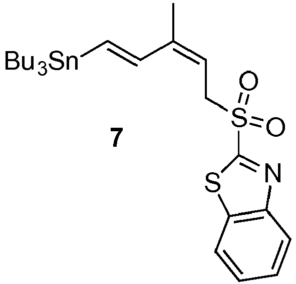 mit Wasserstoff und einem Peroxy-Molybdat (VI)-Reagenz;
(c) Umsetzen des Sulfons mit der Formel 7 jeweils mit R- oder S-Enantiomer von Aldehyd 8, 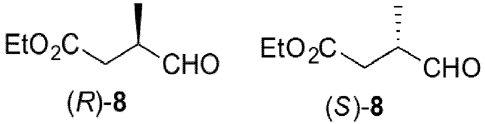 in Gegenwart von Base durch Julia-Kocienski-Olefinierung;
um das wie in Anspruch 2 definierte Stannan mit der Formel 9 zu erhalten.

4. Verfahren nach einem der Ansprüche 1 bis 3, wobei die Enatiomere enantiomerenreine Verbindungen sind.

5. Enantiomerenverbindung von 9-cis-13,14-Dihydro-Retinsäure oder einer Estervorstufe davon, wobei die Verbindung aus der Gruppe bestehend aus
(*R*)-9-cis-13,14-Dihydro-Retinsäure,
(*S*)-9-cis-13,14-Dihydro-Retinsäure,
(9Z,13R)-Ethyl-13,14-Dihydro-Retinoat((R) -4) und
(9Z, 13R)-Ethyl-13,14-Dihydro-Retinoat((S) -4) und
Solvate, feste Formen und gegebenenfalls Salze davon
ausgewählt ist.

6. Enantiomerenverbindung von 9-cis-13,14-Dihydro-Retinsäure,
wobei die Verbindung aus der Gruppe bestehend aus
(*R*)-9-cis-13,14-Dihydro-Retinsäure,
(*S*)-9-cis-13,14-Dihydro-Retinsäure und
Solvate, feste Formen und Salze davon
ausgewählt ist.

7. Enantiomerenverbindung (R)-9-cis-13,14-Dihydro-Retinsäure oder ein Solvat, feste Form oder Salz davon.

8. Enantiomerenverbindung nach einem der Ansprüche 4 bis 6, wobei die Verbindung in einer enantiomerenreine Form vorliegt.

9. Pharmazeutische Zusammensetzung umfassend eine enantiomerenreine Verbindung ausgewählt aus den Enantiomerenverbindungen nach Anspruch 5, Solvaten, festen Formen und gegebenenfalls Salzen davon, in einem pharmazeutisch verträglichen Träger.

10. Pharmazeutische Zusammensetzung umfassend eine enantiomerenreine Verbindung von 9-cis-13,14-Dihydro-Retinsäure in einem pharmazeutisch verträglichen Träger,
wobei die Verbindung aus der Gruppe bestehend aus
(*R*)-9-cis-13,14-Dihydro-Retinsäure,
(*S*)-9-cis-13,14-Dihydro-Retinsäure,
Solvate, Salze und feste Formen davon
ausgewählt ist.

11. Enantiomerenverbindung von 9-cis-13,14-Dihydro-Retinsäure,
wobei die Verbindung aus der Gruppe bestehend aus
(R)-9-cis-13,14-Dihydro-Retinsäure,
(S)- 9-cis-13,14-Dihydro-Retinsäure
ausgewählt ist,
oder eine pharmazeutische Zusammensetzung nach Anspruch 8,
zur Verwendung in einem therapeutischen Verfahren.

12. Die Enantiomerenverbindung oder die pharmazeutische Zusammensetzung zur Verwendung nach Anspruch 11, zur Verwendung bei der Behandlung einer psychiatrischen Störung.

13. Die Enantiomerenverbindung oder die pharmazeutische Zusammensetzung zur Verwendung nach Anspruch 11, zur Verwendung bei der Vorbeugung und/oder Behandlung von Gedächtnisstörung oder zur Verwendung bei Verbesserung von Gedächtnisleistung, wobei das Gedächtnis vorzugsweise Arbeitsgedächtnis ist, oder zur Verwendung bei der Vorbeugung und/oder Behandlung von beeinträchtigten kognitiven Funktionen oder beeinträchtigter Lernfähigkeit, oder zur Verwendung bei der Behandlung von Depression.

14. (*R*)-9-cis-13,14-Dihydro-Retinsäure zur Verwendung bei der Vorbeugung und/oder Behandlung von Gedächtnisstörung oder zur Verwendung bei Verbesserung von Gedächtnisleistung, wobei das Gedächtnis vorzugsweise Arbeitsgedächtnis ist, oder zur Verwendung bei der Vorbeugung und/oder Behandlung von beeinträchtigten kognitiven Funktionen oder beeinträchtigter Lernfähigkeit, oder zur Verwendung bei der Behandlung von Depression.

15. Funktionelles Nahrungsmittel oder Nahrungsergänzungsmittel umfassend eine oder mehrere enantiomerenreine Verbindung(en) nach einem der Ansprüche 4-6.

## Revendications

1. Procédé énantiosélectif pour la préparation d'un composé énantiomère R ou S choisi dans le groupe constitué par l'acide R ou S 9-cis-13,14-dihydrorétinoïque ou de leurs esters, répondant à la formule générale I, un solvate et, le cas échéant, un sel celui-ci, ledit procédé comprenant 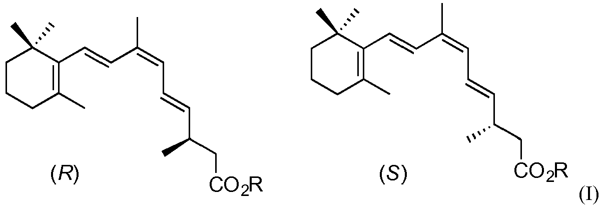 où, dans ladite formule, R est choisi parmi H ou éthyle ;
- fournir l'isomère géométrique respectif 9Z, 11E de l'énantiomère R ou S de l'iodure de triényle de Formule 3 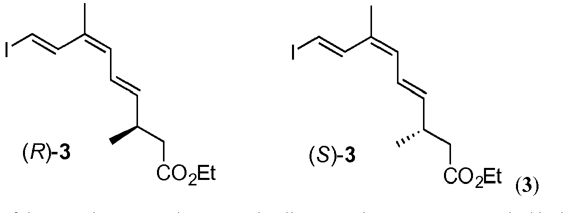
- faire réagir, respectivement, lesdits énantiomères R ou S de l'iodure de triényle de Formule **3** avec de l'acide boronique de Formule **2** par couplage de Suzuki pour obtenir ledit composé sous forme de R ou S 9-cis-13,14-dihydrorétinoate d'éthyle de formule (I) ; 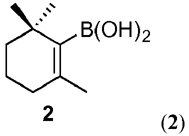
- éventuellement saponifier ledit R ou S 9-cis-13,14-dihydrorétinoate d'éthyle pour obtenir ledit composé sous forme d'acide R ou S 9-cis-13,14-dihydrorétinoïque, respectivement ;
- éventuellement transformer ledit composé en un solvate ou, le cas échéant, en son sel.

2. Le procédé selon la revendication 1, dans lequel
l'énantiomère R ou S de l'iodure de triényle de Formule 3 est préparé à partir du stannane de formule **9** 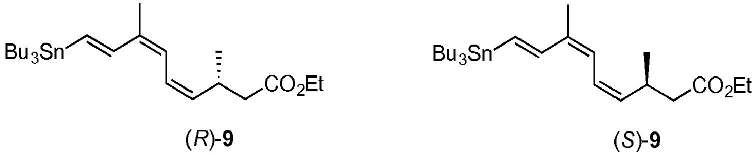 avec une solution d'iode dans un solvant, de préférence CH₂Cl₂, par échange Sn-I et isomérisation favorisée par l'iode du diène 9Z, 11Z en l'isomère géométrique 9Z, 11E désiré.

3. Le procédé selon la revendication 2, comprenant en outre les étapes consistant à :
(a) transformer le stannyldiénol de Formule **5** 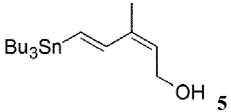 par réaction de Mitsunobu avec le thiol correspondant en sulfure de benzothiazolyle et d'allyle ayant la Formule **6** 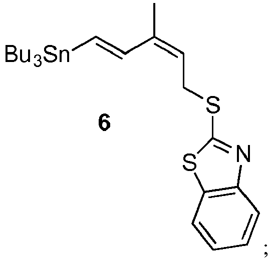
(b) oxyder du sulfure de benzothiazolyle et d'allyle ayant la Formule 6 en la sulfone correspondante ayant la Formule 7 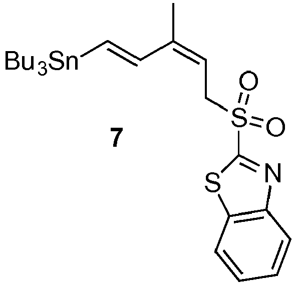 avec de l'hydrogène et un réactif peroxymolybdate (VI) ;
(c) faire réagir la sulfone ayant la Formule 7 avec l'énantiomère R ou S de l'aldéhyde **8,** respectivement, 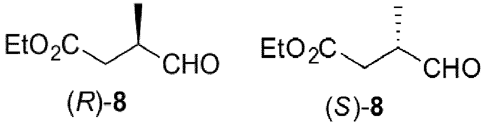 en présence de base par l'oléfination de Julia-Kocienski ;
pour obtenir le stannane de Formule **9** tel que défini dans la revendication 2.

4. Le procédé selon l'une quelconque des revendications 1 à 3, dans lequel les énantiomères sont des composés énantiomériquement purs.

5. Composé énantiomère d'acide 9-*cis*-13,14-dihydrorétinoïque ou d'un ester précurseur de celui-ci où ledit composé est choisi dans le groupe constitué par
l'acide (*R*)-9-*cis*-13,14-dihydrorétinoïque,
l'acide (S)-9-*ci*s-13,14-dihydrorétinoïque,
(9Z,13R)-13,14-dihydrorétinoate d'éthyle ((R)-4) et
(9Z,13R)-13,14-dihydrorétinoate d'éthyle ((S)-4), et
les solvates, les formes solides et, le cas échéant, les sels de ceux-ci.

6. Composé énantiomère d'acide 9-*cis*-13,14-dihydrorétinoïque
où ledit composé est choisi dans le groupe constitué par
l'acide (*R*)-9-*cis*-13,14-dihydrorétinoïque,
l'acide (*S*)-9-*cis*-13,14-dihydrorétinoïque, et
les solvates, les formes solides et les sels de ceux-ci.

7. Composé énantiomère d'acide (*R*)-9-*cis*-13,14-dihydrorétinoïque ou un solvate, une forme solide ou un sel de celui-ci.

8. Le composé énantiomère selon l'une quelconque des revendications 4 à 6, où le composé est sous une forme énantiomériquement pure.

9. Composition pharmaceutique comprenant un composé énantiomériquement pur choisi parmi les composés énantiomères selon la revendication 5, les solvates, les formes solides et, le cas échéant, les sels de ceux-ci,
dans un véhicule pharmaceutiquement acceptable.

10. Composition pharmaceutique comprenant un composé énantiomériquement pur d'acide 9-*cis*-13,14-dihydrorétinoïque,
où ledit composé est choisi dans le groupe constitué par
l'acide (*R*)-9-*cis*-13,14-dihydrorétinoïque,
l'acide (*S*)-9-*cis*-13,14-dihydrorétinoïque, et
les solvates, les sels et les formes solides de ceux-ci,
dans un véhicule pharmaceutiquement acceptable.

11. Composé énantiomère d'acide 9-cis-13,14-dihydrorétinoïque,
où ledit composé est choisi dans le groupe constitué par
l'acide (*R*)-9-*cis*-13,14-dihydrorétinoïque,
l'acide (*S*)-9-*cis*-13,14-dihydrorétinoïque,
ou une composition pharmaceutique selon la revendication 8,
pour utilisation dans une méthode thérapeutique.

12. Le composé énantiomère ou la composition pharmaceutique pour utilisation selon la revendication 11, pour utilisation dans le traitement d'un trouble psychiatrique.

13. Le composé énantiomère ou la composition pharmaceutique pour utilisation selon la revendication 11, pour utilisation dans la prévention et/ou dans le traitement des troubles de la mémoire ou pour améliorer les performances de la mémoire, où de préférence ladite mémoire est la mémoire de travail, ou pour utilisation dans la prévention et/ou dans le traitement des troubles des fonctions cognitives ou d'apprentissage, ou pour le traitement de la dépression.

14. L'acide (*R*)-9-*cis*-13,14-dihydrorétinoïque pour utilisation dans la prévention et/ou dans le traitement des troubles de la mémoire ou pour améliorer les performances de la mémoire, où de préférence ladite mémoire est la mémoire de travail, ou pour utilisation dans la prévention et/ou dans le traitement des troubles des fonctions cognitives ou d'apprentissage, ou pour le traitement de la dépression.

15. Aliment fonctionnel ou complément alimentaire comprenant un ou plusieurs composé(s) énantiomériquement pur(s) selon l'une quelconque des revendications 4 à 6.
